# EUROPEAN PATENT SPECIFICATION

(11) **EP 4 339 184 B1**
(45) Date of publication and mention of the grant of the patent: **12.08.2026**
(21) Application number: 22806760.9
(22) Date of filing: 10.05.2022
(51) Int. Cl.: C07C 233/51, A61K 31/192, A61P 31/12, C07D 307/16

(54) **ALKYL CARBOXYLIC ACID COMPOUNDS AND APPLICATION THEREOF**
ALKYLCARBONSÄUREVERBINDUNGEN UND DEREN VERWENDUNG
COMPOSÉS D'ACIDE ALKYLCARBOXYLIQUE ET LEUR APPLICATION

(30) Priority: 14.05.2021 CN 202110530275; 02.12.2021 CN 202111463413
(43) Date of publication of application: 20.03.2024
(73) Proprietor: Medshine Discovery Inc., Nanjing, Jiangsu 210032 (CN)
(72) Inventor: LUO, Yunfu, Shanghai 200131 (CN); GE, Weizhi, Shanghai 200131 (CN); LI, Shaolong, Shanghai 200131 (CN); ZHANG, Guoli, Shanghai 200131 (CN); CHEN, Shuhui, Shanghai 200131 (CN)
(74) Representative: Dehns
(86) International application number: PCT/CN2022/092034
(87) International publication number: WO 2022/237797

(56) References cited:
- WO-A1-2012/139888
- WO-A1-2018/153899
- CN-A- 102 712 577
- CN-A- 103 796 989
- DATABASE CAPLUS [online] 1 January 2012 (2012-01-01), BAYER INTELLECTUAL PROPERTY GMBH ET AL: "Preparation of branched 3-phenylpropionic acid derivatives as guanylate cyclase activators - WO 2012139888 A1", XP093257703, Database accession no. 2012:1519561, 2012:1514014
- DATABASE CAPLUS [online] 1 January 2012 (2012-01-01), BAYER INTELLECTUAL PROPERTY GMBH ET AL: "Preparation of branched 3-phenylpropionic acid derivatives as guanylate cyclase activators - WO 2012139888 A1", XP093257713, Database accession no. 2012:1519561, 2012:1514014

## Description

### TECHNICAL FIELD

The present disclosure relates to a series of alkyl carboxylic acid compounds and to the compounds for use in treating chronic kidney disease, and specifically relates to a compound of formula (II) and a pharmaceutically acceptable salt thereof.

### BACKGROUND

Soluble guanylate cyclase (sGC) is an enzyme-linked receptor for the second messenger nitric oxide (NO) and is widely present in various cell types, including muscle, epithelial, neuronal, and endothelial cells. sGC is a heterodimer consisting of either an α1 or α2 subunit bound to a β1 subunit. The β1 subunit contains a heme cofactor and serves as the key signal transduction enzyme in the NO-sGC-cGMP signaling pathway. Under physiological conditions, NO binds to the heme cofactor of sGC, and upon activation, it catalyzes the conversion of guanosine-5'-triphosphate (GTP) to cyclic guanosine monophosphate (cGMP).

cGMP is a significant secondary messenger molecule. By activating a variety of downstream effector molecules, such as phosphodiesterase (PDE), cyclic nucleotide-gated ion channel (CNG), and protein kinase (PKG), it subsequently triggers a series of downstream cascade reactions and plays critical physiological roles in the gastrointestinal system, circulatory system, and nervous system. Functions include promoting vasodilation and smooth muscle relaxation, inhibiting platelet aggregation, vascular remodeling, apoptosis, and inflammation, as well as participating in neurotransmission. Therefore, sGC stimulators can serve as potential therapeutic agents for treating cardiovascular diseases (heart failure, pulmonary arterial hypertension, angina, myocardial infarction) and fibrotic diseases (renal fibrosis, systemic sclerosis). Under the aforementioned pathological conditions, prolonged oxidative stress can lead to the oxidation of the heme cofactor in sGC (from a ferrous state to a ferric state), which causes the sGC enzyme unresponsive to NO activation and may exacerbate the progression of the disease. It further contributes to diseases such as endothelial dysfunction, atherosclerosis, hypertension, stable or unstable angina, thrombosis, myocardial infarction, stroke, or worsening of erectile dysfunction. Therefore, activating the oxidized sGC to produce cGMP makes the treatment and/or prevention of such diseases possible.

sGC activators are independent of NO and also independent of the heme cofactor, and are capable of directly activating the sGC-cGMP signaling pathway. sGC activators have the potential to offer benefits in many diseases caused by defective NO signaling pathway, particularly following oxidative stress.

Addressing the current market and clinical unmet needs for such soluble guanylate cyclase stimulators, the present disclosure provides a new class of compounds. These compounds can act as activators of soluble guanylate cyclase, exhibiting excellent *in vitro* stimulatory activity on soluble guanylate cyclase and favorable pharmacokinetic properties.

WO 2012/139888 A1 discloses branched 3-phenylpropionic acid derivatives as guanylate cyclase activators.

### CONTENT OF THE PRESENT INVENTION

The present disclosure provides a compound of formula (II) or a pharmaceutically acceptable salt thereof, 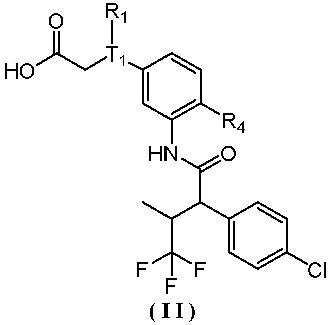 wherein
R₁ is selected from 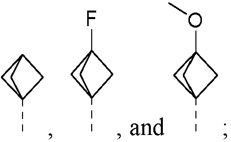
R₄ is selected from halogen and C₁₋₃ alkyl;
T₁ is selected from CH and N.

In some embodiments of the present disclosure, the R₄ is selected from F, Cl, and methyl, and other variables are as defined in the present disclosure.

The present disclosure provides a compound of formula (I) or a pharmaceutically acceptable salt thereof, 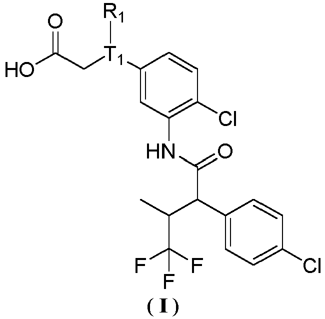 wherein
R₁ is selected from 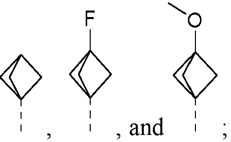
T₁ is selected from CH and N.

There are still some embodiments of the present disclosure which are obtained by any combination of the above variables.

The present disclosure also provides a compound of formula (II-1) or a pharmaceutically acceptable salt thereof, 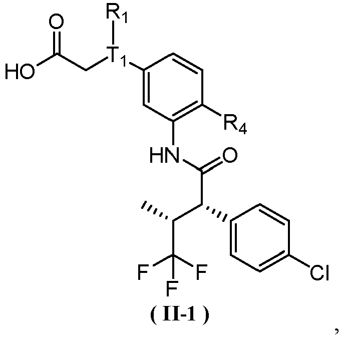 wherein R₁, R₄, and T₁ are as defined in the present disclosure.

The present disclosure also provides the following compounds or pharmaceutically acceptable salts thereof:

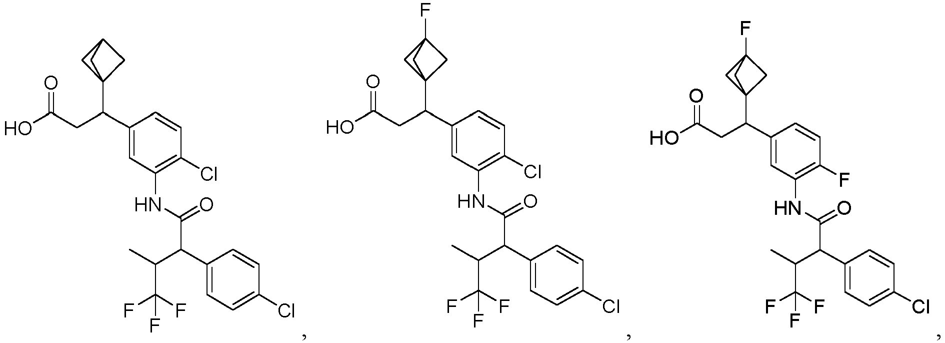

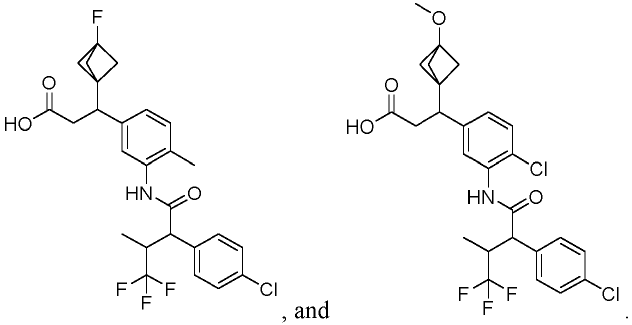

In some embodiments of the present disclosure, the compound is selected from:

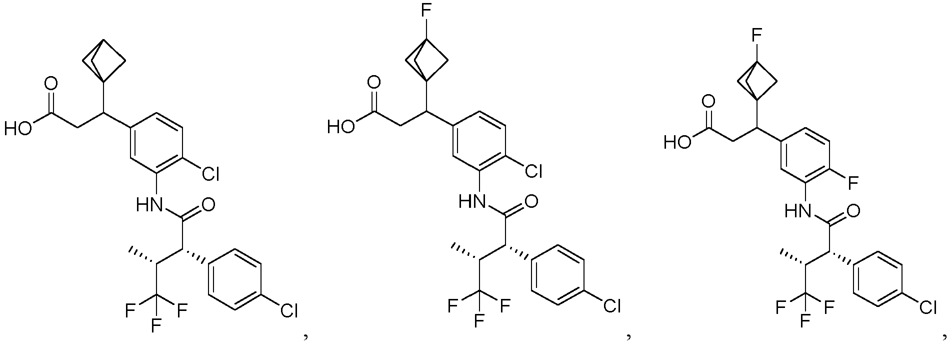

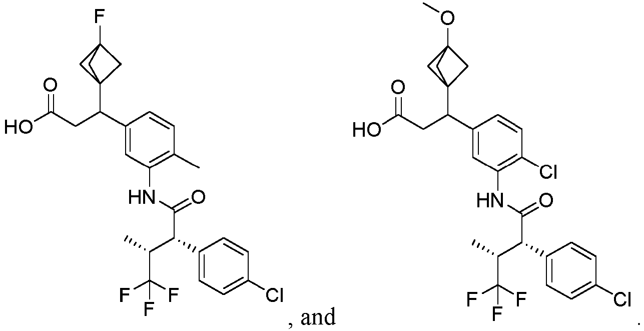

In some embodiments of the present disclosure, the compound is selected from: 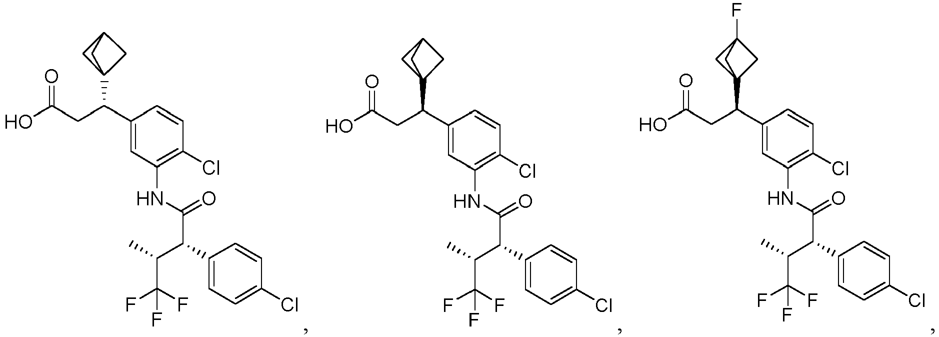 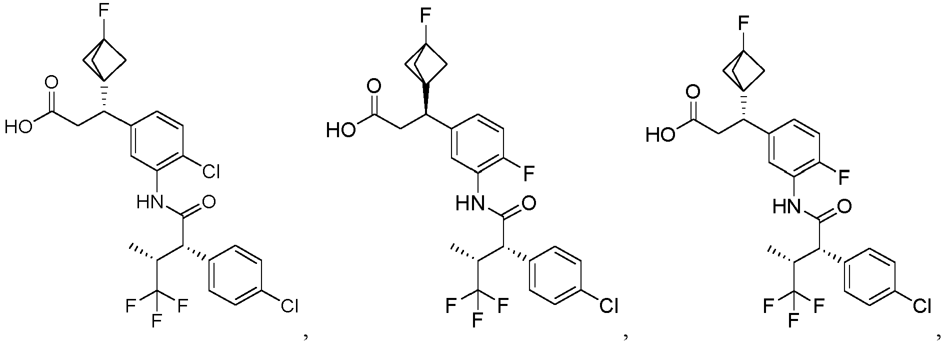 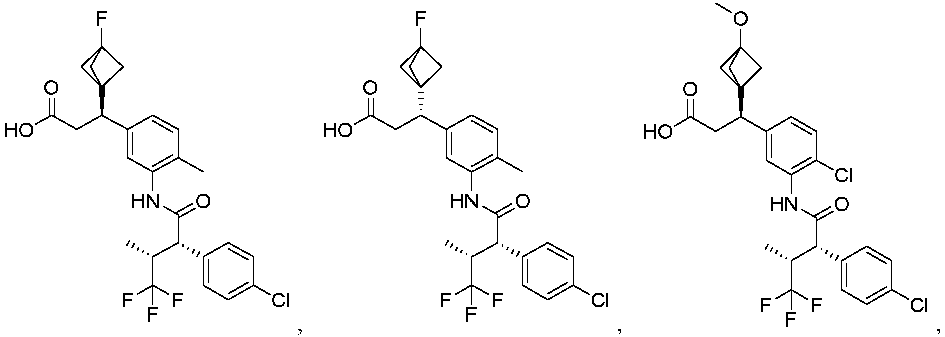 and

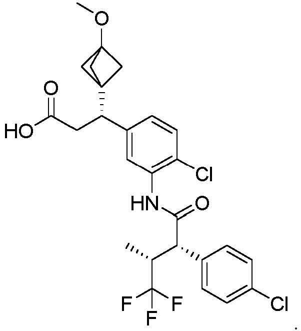

The present disclosure also provides the compound or the pharmaceutically acceptable salt thereof as herein described for use in treating chronic kidney disease.

### Definition and description

Unless otherwise specified, the following terms and phrases when used herein have the following meanings. A specific term or phrase should not be considered indefinite or unclear in the absence of a particular definition, but should be understood in the ordinary sense. When a trading name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction, or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by bringing the compound into contact with a sufficient amount of base in a pure solution or a suitable inert solvent. When the compound of the present disclosure contains a relatively basic functional group, an acid addition salt can be obtained by contacting the compound with a sufficient amount of acid in a pure solution or a suitable inert solvent. Certain specific compounds of the present disclosure contain both basic and acidic functional groups, thus can be converted to any base or acid addition salt.

As for the pharmaceutically acceptable salt in the present disclosure, the term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by bringing the compound into contact with a sufficient amount of base in a pure solution or a suitable inert solvent. When the compound of the present disclosure contains a relatively basic functional group, an acid addition salt can be obtained by contacting the compound with a sufficient amount of acid in a pure solution or a suitable inert solvent. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by conventional chemical method. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplates all such compounds, including *cis* and *trans* isomers, (-)- and (+)-enantiomers, (*R*)- and (*S*)-enantiomers, diastereoisomers, (*D*)-isomers, (*L*)-isomers, racemic, and other mixtures thereof, such as enantiomer or diastereomer enriched mixtures, all of which are within the scope of the present disclosure. Additional asymmetric carbon atoms may be present in substituents such as alkyl. All these isomers and their mixtures are included within the scope of the present disclosure.

Unless otherwise specified, the term "enantiomer" or "optical isomer" refers to stereoisomers that are mirror images of each other.

Unless otherwise specified, the term "diastereomer" refers to a stereoisomer in which a molecule has two or more chiral centers and the relationship between the molecules is not mirror images.

Unless otherwise specified, "(+)" refers to dextrorotation, "(-)" refers to levorotation, and "(±)" refers to racemic.

Unless otherwise specified, the absolute configuration of a stereogenic center is represented by a wedged solid bond ( 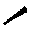 ) and a wedged dashed bond ( 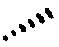 ), and the relative configuration of a stereogenic center is represented by a straight solid bond ( 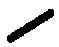 ) and a straight dashed bond ( 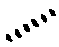 ), a wave line ( 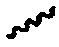 ) is used to represent a wedged solid bond ( 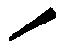 ) or a wedged dashed bond ( 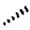 ), or the wave line ( 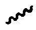 ) is used to represent a straight solid bond ( 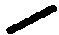 ) and a straight dashed bond ( 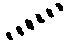 ).

Unless otherwise specified, when a group has one or more linkable sites, any one or more sites of the group can be linked to other groups through chemical bonds. The chemical bond between the site and other groups can be represented by a straight solid bond ( 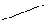 ), a straight dashed bond ( 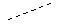 ), or a wavy line ( 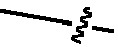 ). For example, the straight solid bond in - OCH₃ means that it is linked to other groups through the oxygen atom in the group; the straight dashed bond in 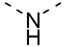 means that it is linked to other groups through the two ends of nitrogen atom in the group; the wave line in 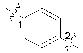 means that the phenyl group is linked to other groups through carbon atoms at position 1 and position 2.

Unless otherwise specified, the term "C₁₋₃ alkyl" refers to a linear or branched saturated hydrocarbon group consisting of 1 to 3 carbon atoms. The C₁₋₃ alkyl includes C₁₋₂ and C₂₋₃ alkyl, etc.; it can be monovalent (such as methyl), divalent (such as methylene), or multivalent (such as methine). Examples of C₁₋₃ alkyl include, but are not limited to, methyl (Me), ethyl (Et), propyl (including *n*-propyl and isopropyl), etc.

Unless otherwise specified, the term "halogen element" or "halogen" by itself or as part of another substituent refers to fluorine, chlorine, bromine, or iodine atom.

The compounds of the present disclosure may exist in specific forms. Unless otherwise specified, the term "tautomer" or "tautomeric form" means that at room temperature, the isomers of different functional groups are in dynamic equilibrium and can be transformed into each other quickly. If tautomers possibly exist (such as in solution), the chemical equilibrium of tautomers can be reached. For example, proton tautomer (also called prototropic tautomer) includes interconversion through proton migration, such as keto-enol isomerization and imine-enamine isomerization. Valence tautomer includes some recombination of bonding electrons for mutual transformation. A specific example of keto-enol tautomerization is the tautomerism between two tautomers of pentane-2,4-dione and 4-hydroxypent-3-en-2-one.

Unless otherwise specified, the terms "enriched in one enantiomer", or "enriched in enantiomers" refer to the content of one of the enantiomers is less than 100%, and the content of the enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "enantiomeric excess" refers to the difference between the relative percentages of two enantiomers. For example, if the content of one enantiomer is 90%, and the content of the other erenantiomer is 10%, the enantiomer excess (ee value) is 80%.

Optically active (*R*)- and (*S*)-isomers, or *D* and *L* isomers can be prepared using chiral synthesis, chiral reagents, or other conventional techniques. If one kind of enantiomer of certain compound of the present disclosure is to be obtained, it can be obtained by asymmetric synthesis or derivative action of chiral auxiliary, wherein the resulting diastereomeric mixture is separated and the auxiliary group is cleaved to provide the pure desired enantiomer. Alternatively, when the molecule contains a basic functional group (such as amino) or an acidic functional group (such as carboxyl), a salt of a diastereoisomer is formed with an appropriate optically active acid or base, and then diastereomeric resolution is performed by conventional methods known in the art, and then the pure enantiomer is recovered. In addition, the enantiomer and the diastereoisomer are generally separated through chromatography which uses a chiral stationary phase and optionally combines with a chemical derivative method (such as carbamate generated from amine).

The compound of the present disclosure may contain an unnatural proportion of atomic isotope at one or more atoms that constitute the compound. For example, the compound can be radiolabeled with a radioactive isotope, such as tritium (³H), iodine-125 (¹²⁵I), or C-14 (¹⁴C). For another example, deuterated drugs can be formed by replacing hydrogen with deuterium, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, etc. All isotopic variations of the compound of the present disclosure, whether radioactive or not, are encompassed within the scope of the present disclosure.

The term "optional" or "optionally" means that the subsequent event or condition may occur but not requisite, that the term includes the instance in which the event or condition occurs and the instance in which the event or condition does not occur.

Unless otherwise specified, when a group has one or more linkable sites, any one or more sites of the group can be linked to other groups through chemical bonds. When the linking site of the chemical bond is not positioned, and there is an H atom at the linkable site, then the number of H atoms at the site will decrease correspondingly with the number of the chemical bonds linking thereto so as to meet the corresponding valence. The chemical bond between the site and other groups can be represented by a straight solid bond ( 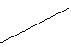 ), a straight dashed bond ( 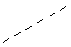 ), or a wavy line ( 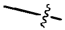 ). For example, the straight solid bond in -OCH₃ means that it is linked to other groups through the oxygen atom in the group; the straight dashed bond in 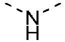 means that it is linked to other groups through the two ends of nitrogen atom in the group; the wave line in 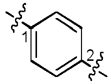 means that the phenyl group is linked to other groups through carbon atoms at position 1 and position 2.

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include but are not limited to the examples of the present disclosure.

The structure of the compounds of the present disclosure can be confirmed by conventional methods known to those skilled in the art, and if the present disclosure involves an absolute configuration of a compound, then the absolute configuration can be confirmed by means of conventional techniques in the art. For example, in the case of single crystal X-ray diffraction (SXRD), diffraction intensity data are collected from the cultured single crystal using a Bruker D8 venture diffractometer with CuKα radiation as the light source and scanning mode: φ/ω scan, and after collecting the relevant data, the crystal structure is further analyzed by direct method (Shelxs97), so that the absolute configuration can be confirmed.

The solvent used in the present disclosure is commercially available. In the present disclosure, the following abbreviations are used: aq stands for water; eq stands for equivalent or equivalent weight; M stands for mol/L; DMSO stands for dimethyl sulfoxide; EtOH stands for ethanol; CBz stands for benzyloxycarbonyl, which is an amine protecting group; Boc stands for tert-butoxycarbonyl, which is an amine protecting group; r.t. stands for room temperature; Boc₂O stands for di-tert-butyl dicarbonate; TFA stands for trifluoroacetic acid; IPAm stands for isopropylamine.

The compounds of the present disclosure are named according to the conventional naming principles in the art or by ChemDraw^{®} software, and the commercially available compounds use the supplier catalog names.

### Technical effect

The compounds of the present disclosure exhibit significant *in vitro* stimulatory activity on guanylate cyclase and possess excellent pharmacokinetic properties as well as good hepatocyte stability.

### DETAILED DESCRIPTION OF THE PREFERRED EMBODIMENT

The present disclosure is described in detail by the examples below. The invention is defined by the claims. The present disclosure has been described in detail herein, and its specific examples have also been disclosed.

In the following Examples, compounds WX004 - WX015 are according to the invention, whereas the compounds WX001 - WX003 and WX016 - WX019 are not according to the invention.

### Example 1

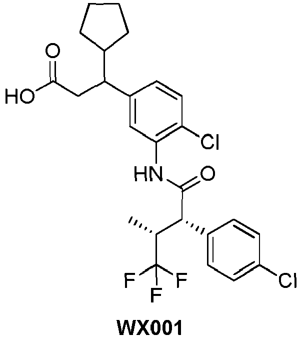

### Synthetic route:

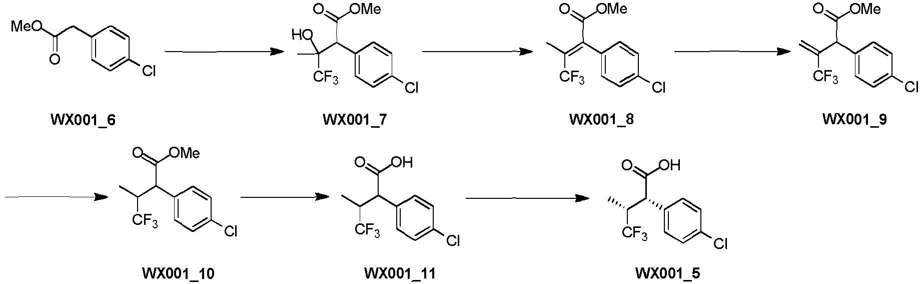

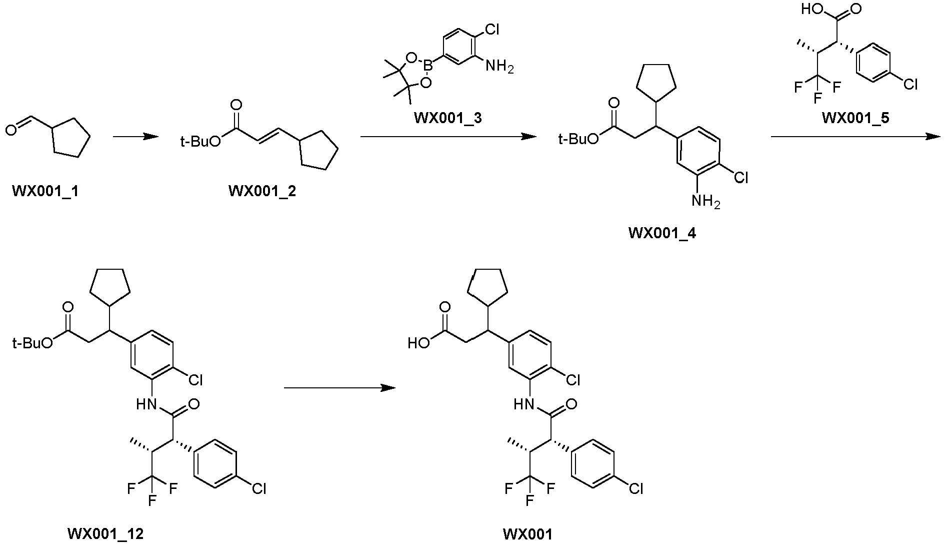

### Step 1: Synthesis of compound WX001_2

tert-Butyl diethylphosphonoacetate (5.14 g, 20.38 mmol) was dissolved in tetrahydrofuran (30 mL) at room temperature under nitrogen atmosphere. The reaction system was cooled to 0°C, and potassium tert-butoxide (2.52 g, 22.48 mmol) was added thereto. After the reaction mixture was stirred for 30 minutes, **WX001_1** (2 g, 20.38 mmol) was slowly added dropwise thereto. Once the addition was complete, the reaction system returned to room temperature and was stirred for 12 hours. After the reaction was complete, the reaction mixture was diluted with water (50 mL), and then extracted with ethyl acetate (60 mL × 3). The organic phases were combined. The organic phase was washed with saturated brine (60 mL × 2), dried over anhydrous sodium sulfate, filtered, and then concentrated under reduced pressure to remove the solvent. The resulting crude product was separated by column chromatography (eluent: petroleum ether/ethyl acetate = 1/0 to 1/1, v/v) to obtain compound **WX001_2**. ¹H NMR (400 MHz, DMSO_*d*₆) δ: 6.75 (dd, *J*=8.0, 15.5 Hz, 1H), 5.75-5.65 (m, 1H), 2.62 -2.52 (m, 1H), 1.83-1.72 (m, 2H), 1.65-1.53 (m, 4H), 1.42 (s, 9H), 1.36-1.28 (m, 2H).

### Step 2: Synthesis of compound WX001_4

2,2'-Bis(diphenylphosphino)-1,1'-binaphthalene (18.24 mg, 29.29 µmol) and chloro(1,5-cyclooctadiene)rhodium(I) dimer (62.80 mg, 127.37 µmol) were dissolved in tetrahydrofuran (10 mL) at room temperature under nitrogen atmosphere. The reaction system was stirred at room temperature for 15 minutes. Compound **WX001_3** (1.42 g, 5.60 mmol) was dissolved in isopropanol (5 mL) and tetrahydrofuran (10 mL). Compound **WX001_2** (1.00 g, 5.09 mmol), potassium hydroxide (343.00 mg, 6.11 mmol), and 1,5-cyclooctadiene (55.11 mg, 509.46 µmol) were sequentially added thereto. The reaction system was heated to 60°C, added with the prepared catalyst, and stirred at 60°C for 9 hours. After the reaction was complete, the reaction mixture was cooled to room temperature, diluted with water (150 mL), and then extracted with ethyl acetate (150 mL × 3). The organic phases were combined. The organic phase was washed with saturated brine (100 mL × 2), dried over anhydrous sodium sulfate, filtered, and then concentrated under reduced pressure to remove the solvent. The resulting crude product was separated by column chromatography (eluent: petroleum ether/ethyl acetate = 1/0 to 1/1, v/v) to obtain compound **WX001_4**. ¹H NMR (400 MHz, CDCl₃) δ: 7.12 (d, *J*=8.1 Hz, 1H), 6.60 (d, *J*=2.0 Hz, 1H), 6.52 (dd, *J*=2.0, 8.3 Hz, 1H), 3.98 (s, 2H), 2.73-2.61 (m, 2H), 2.45-2.35 (m, 1H), 1.97-1.78 (m, 2H), 1.70-1.61 (m, 1H), 1.59-1.49 (m, 2H), 1.45-1.35 (m, 2H), 1.28-1.25 (m, 9H), 1.08-0.96 (m, 1H), 0.91-0.82 (m, 1H).

### Step 3: Synthesis of compound WX001_7

Diisopropylamine (31.52 g, 311.45 mmol, 44.02 mL) was placed in a dry reaction flask at room temperature under nitrogen atmosphere. Tetrahydrofuran (350 mL) was added to dissolve the compound. The reaction system was cooled to -70°C, and a solution of n-butyllithium in hexane (130.00 mL, 324.99 mmol, 2.5 M) was added thereto. The reaction system was stirred at -70°C for 30 minutes, then warmed to -40°C, and stirred for another 30 minutes. The reaction system was cooled to -70°C, and **WX001_6** (50 g, 270.83 mmol) dissolved in tetrahydrofuran (120 mL) was added thereto. The reaction system was stirred at -70°C for 1 hour. Trifluoroacetone (45.52 g, 406.24 mmol) dissolved in tetrahydrofuran (150 mL) was then added, and the reaction system was stirred at -70°C for another 2 hours. After the reaction was complete, the reaction system was warmed to 0°C, added with 120 mL of 5 N dilute hydrochloric acid to quench the reaction, and then stirred at room temperature for 30 minutes. The reaction mixture was concentrated under reduced pressure to remove most of the organic solvent, and the residue was diluted with ethyl acetate (300 mL). The phases were separated, and the organic phase was collected. The aqueous phase was extracted with ethyl acetate (200 mL × 3), and the organic phases were combined. The organic phase was washed with saturated brine (200 mL × 2), dried over anhydrous sodium sulfate, filtered, and concentrated under reduced pressure to remove the solvent to obtain compound **WX001_7.**

### Step 4: Synthesis of compound WX001_8

**WX001_7** (80.35 g, 270.84 mmol) was dissolved in pyridine (160 mL) at room temperature under nitrogen atmosphere. To the reaction system at room temperature was added phosphorus oxychloride (49.83 g, 325.01 mmol). The reaction system was stirred at 110°C for 12 hours. After the reaction was complete, the reaction system was cooled to room temperature, poured into 200 mL of water to quench the reaction, added with 6 N dilute hydrochloric acid to adjust the pH to around 3, and diluted with dichloromethane (400 mL). The phases were separated, and the organic phase was collected. The aqueous phase was extracted with dichloromethane (300 mL × 3), and the organic phases were combined. The organic phase was washed with saturated brine (200 mL × 2), dried over anhydrous sodium sulfate, filtered, and then concentrated under reduced pressure to remove the solvent. The resulting crude product was separated by column chromatography (eluent: petroleum ether/ethyl acetate = 1/0 to 9/1, v/v) to obtain a mixture of compounds **WX001_8** and **WX001_9**. The mixture was then separated by column chromatography (eluent: petroleum ether/ethyl acetate = 1/0 to 9/1, v/v) to obtain compound **WX001_8.**

### Step 5: Synthesis of compound WX001_9

Diisopropylamine (7.99 g, 78.95 mmol) was placed in a dry reaction flask and dissolved in tetrahydrofuran (50 mL) at room temperature under nitrogen atmosphere. The reaction system was replaced with nitrogen three times and then cooled to -60°C. A solution of n-butyllithium in hexane (71.77 mmol, 28.71 mL, 2.5 M) was slowly added thereto. The reaction system was warmed to -40°C and stirred at -40°C for 30 minutes. Afterwards, the reaction system was cooled to -65°C, and **WX001_8** (10 g, 35.89 mmol) dissolved in tetrahydrofuran (50 mL) was added dropwise thereto. The reaction system was stirred at - 65°C for another 1.5 hours. After the reaction was complete, a solution of acetic acid (8.62 g, 143.55 mmol) in tetrahydrofuran (40 mL) was added to the reaction system to quench the reaction. The reaction mixture was then stirred at room temperature for 2 hours. The reaction mixture was diluted with water (80 mL) and ethyl acetate (80 mL), and then the phases were separated. The organic phase was collected and the aqueous phase was extracted with ethyl acetate (80 mL × 3). The organic phases were then combined. The organic phase was washed with saturated brine (80 mL × 2), dried over anhydrous sodium sulfate, filtered, and then concentrated under reduced pressure to remove the solvent. The resulting crude product was separated by column chromatography (eluent: petroleum ether/ethyl acetate = 1/0 to 9/1, v/v) to obtain compound **WX001_9**. The crude product was directly used in the next step.

### Step 6: Synthesis of compound WX001_10

Pt/C (0.57 g, purity: 5%) was added to a dry hydrogenation flask and wetted with methanol (60 mL) at room temperature under argon atmosphere. **WX001_9** (5.7 g, 20.46 mmol) was then added to the reaction system. The reaction system was stirred at 50°C under hydrogen atmosphere of 345 kPa (50 psi) for 12 hours. Upon completion of the reaction, the reaction system was filtered through diatomite. The filter cake was washed with methanol (60 mL × 5), and the filtrates were combined and concentrated under reduced pressure to obtain the residue. Compound **WX001_10** was obtained.

### Step 7: Synthesis of compound WX001_11

**WX001_10** (5.8 g, 20.66 mmol) was dissolved in methanol (60 mL) at room temperature under nitrogen atmosphere. To the reaction system was then added sodium methoxide (3.35 g, 61.99 mmol). The reaction system was stirred at 80°C for 12 hours. Additional sodium methoxide (1.12 g, 20.66 mmol) was added thereto, and the reaction system was stirred at 80°C for another 12 hours. The reaction mixture was heated to 90°C and stirred for 12 hours. After the reaction was complete, the reaction mixture was cooled to room temperature, concentrated under reduced pressure to remove most of the organic solvent, diluted with water (60 mL), and then extracted with methyl tert-butyl ether (70 mL). The organic phase was discarded, the aqueous phase was added with 3 N dilute hydrochloric acid to adjust the pH to 2 to 3 and extracted with ethyl acetate (80 mL × 3), and the organic phases were combined. The organic phase was washed with saturated brine (50 mL × 2), dried over anhydrous sodium sulfate, filtered, and then concentrated under reduced pressure to remove the solvent. Compound **WX001_11** was obtained.

### Step 8: Synthesis of compound WX001_5

Quinine (3.51 g, 10.83 mmol) was dissolved in ethanol (36 mL) at room temperature under nitrogen atmosphere. The reaction system was heated to 80°C, and **WX001_11** (3.85 g, 14.44 mmol), dissolved in ethanol (19 mL), was gradually added dropwise thereto. The reaction system was stirred at 80°C for 10 minutes. A white solid precipitated. The reaction system was slowly cooled to 0°C and stirred at 0°C for 1 hour. The reaction mixture was filtered, and the filter cake was washed with 5 mL of water. The solid was collected. The collected solid was dissolved in methanol (157 mL) and water (20 mL). The reaction system was then heated to 100°C and stirred for 30 minutes. It was subsequently slowly cooled to room temperature, resulting in the precipitation of a white solid. The reaction mixture was filtered, and the filter cake was washed with 10 mL of water. The resulting filter cake was collected. The filter cake was dissolved in ethanol (120 mL) and water (20 mL). The reaction system was then heated to 100°C and stirred for 30 minutes. It was subsequently slowly cooled to room temperature, resulting in the precipitation of a white solid. The filter cake was washed with 10 mL of water, and the solid was collected. The solid was suspended in water (25 mL) and hydrochloric acid (6 M, 3.25 mL) was added thereto. The reaction system was stirred at room temperature for 2.5 hours. The white solid was then filtered and the filter cake was washed with 1 mL of water. The solid was collected, and the filter cake was concentrated under reduced pressure to remove the solvent. Thus compound **WX001_5** was obtained.

SFC analysis method: column type: Chiralpak IG-3 (100 × 4.6 mm I.D., 3 µm); mobile phase: A: CO₂, B: [MeOH (containing 0.1% IPAm)], gradient: B%: 5% to 40%, 3 minutes. The retention time for **WX001_5** was recorded as 1.196 minutes.

### Step 9: Synthesis of compound WX001_12

Compound **WX001_5** (150 mg, 562.55 µmol) was dissolved in dichloromethane (3 mL) at room temperature under nitrogen atmosphere. N,N-dimethylformamide (411.19 µg, 5.63 µmol) was added thereto, and the reaction system was cooled to 0°C. Oxalyl chloride (107.11 mg, 843.82 µmol) was then added dropwise thereto. The reaction system was stirred at 25°C for 1 hour, and concentrated under reduced pressure to remove the solvent. The residue was dissolved in dichloromethane (3 mL), and a solution of compound **WX001_4** (218.62 mg, 675.06 µmol) in dichloromethane (2 mL) was added dropwise thereto, along with N,N-diisopropylethylamine (145.41 mg, 1.13 mmol). The reaction system was stirred at room temperature for 2 hours. After the reaction was complete, the reaction mixture was concentrated under reduced pressure to remove the solvent. The resulting crude product was separated by column chromatography (eluent: petroleum ether/ethyl acetate = 1/0 to 7/3, v/v) to obtain compound **WX001_12**. ¹H NMR (400 MHz, CDCl₃) δ: 8.17 (d, *J*=2.4 Hz, 1H), 7.58 (d, *J*=5.3 Hz, 1H), 7.42-7.31 (m, 4H), 7.21 (dd, *J*=2.2, 8.3 Hz, 1H), 6.88 (d, *J*=8.1 Hz, 1H), 3.68 (t, *J*=7.8 Hz, 1H), 3.47-3.34 (m, 1H), 2.84-2.76 (m, 1H), 2.74-2.64 (m, 1H), 2.49-2.39 (m, 1H), 2.02-1.92 (m, 1H), 1.90-1.80 (m, 1H), 1.68-1.61 (m, 1H), 1.59-1.49 (m, 2H), 1.41-1.31 (m, 2H), 1.30-1.27 (m, 1H), 1.23 (d, *J*=3.3 Hz, 9H), 1.09-1.01 (m, 1H), 0.96 (d, *J*=7.2 Hz, 3H).

### Step 10: Synthesis of compound WX001

Compound **WX001_12** (150 mg, 262.02 µmol) was dissolved in ethyl acetate (3 mL) in a pre-dried reaction flask at room temperature under nitrogen atmosphere. Hydrochloric acid in ethyl acetate (4 M, 3 mL) was added thereto, and the reaction system was stirred at room temperature for 10 hours. After the reaction was complete, the reaction mixture was concentrated under reduced pressure to remove the solvent. The resulting residue was separated by preparative HPLC (mobile phase: acetonitrile/water; acidic system: 0.04% HCl) to obtain compound **WX001.** MS-ESI m/z: 516.2 [M+H]⁺. ¹H NMR (400MHz, DMSO_*d*₆) δ: 11.92 (s, 1H), 9.81 (s, 1H), 7.51-7.42 (m, 4H), 7.39-7.35 (m, 1H), 7.33 (d, *J*=8.3 Hz, 1H), 7.06-7.00 (m, 1H), 4.12 (d, *J*=10.7 Hz, 1H), 3.41-3.34 (m, 1H), 2.76-2.69 (m, 1H), 2.65 (dd, *J*=4.2, 15.6 Hz, 1H), 2.46-2.38 (m, 1H), 1.96-1.87 (m, 1H), 1.85-1.75 (m, 1H), 1.64-1.31 (m, 4H), 1.27-1.09 (m, 2H), 0.98-0.89 (m, 1H), 0.80 (d, *J*=7.0 Hz, 3H).

### Example 2

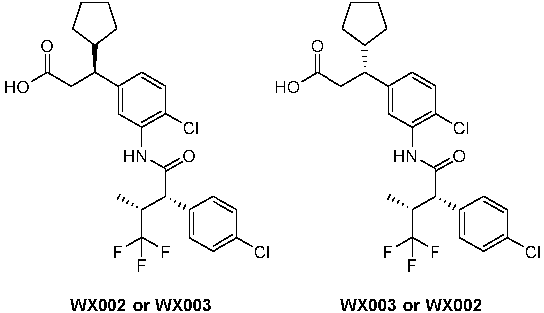

### Step 1: Synthesis of compounds WX002 and WX003

Compound **WX001** was separated by chiral column chromatography (column type: Phenomenex-Cellulose-2 (250 mm * 30 mm, 10 µm); mobile phase: A (CO₂) and B (ethanol containing 0.1% ammonia); gradient: B% = 30% to 30%, 5 minutes) to obtain compounds **WX002** and **WX003,** respectively.

SFC analysis method: column type: Lux Cellulose-2, 50 × 4.6 mm I.D., 3 µm; mobile phase: A: CO₂, B: [EtOH (containing 0.1% IPAm)], gradient: B%: 5% to 50%, 3 minutes.

**WX002** (retention time: 1.154 min): MS-ESI m/z: 516.2 [M+H]⁺. ee value: 98.32%. ¹H NMR (400MHz, DMSO_*d₆*) δ: 9.82 (s, 1H), 7.46 (s, 4H), 7.40-7.28 (m, 2H), 7.03 (d, *J*=8.3 Hz, 1H), 4.12 (d, *J*=10.1 Hz, 1H), 3.45-3.41 (m, 1H), 2.72-2.60 (m, 2H), 2.45-2.30 (m, 1H), 1.99-1.74 (m, 2H), 1.65-1.32 (m, 4H), 1.29-1.08 (m, 2H), 0.93 (s, 1H), 0.80 (d, *J*=6.4 Hz, 3H).

**WX003** (retention time: 1.416 min): MS-ESI m/z: 516.2 [M+H]⁺. ee value: 95.24%. ¹H NMR (400MHz, DMSO_*d*₆) δ: 9.82 (s, 1H), 7.46 (s, 4H), 7.40-7.28 (m, 2H), 7.03 (d, *J*=8.3 Hz, 1H), 4.12 (d, *J*=10.1 Hz, 1H), 3.58-3.47 (m, 1H), 2.72-2.60 (m, 2H), 2.45-2.30 (m, 1H), 1.99-1.74 (m, 2H), 1.65-1.32 (m, 4H), 1.29-1.08 (m, 2H), 0.93 (s, 1H), 0.80 (d, *J*=6.4 Hz, 3H).

### Example 4

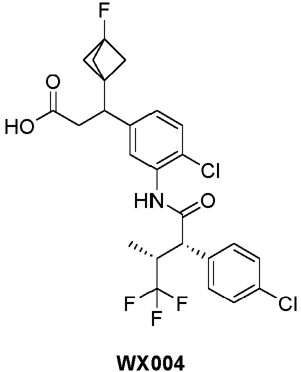

### Synthetic route:

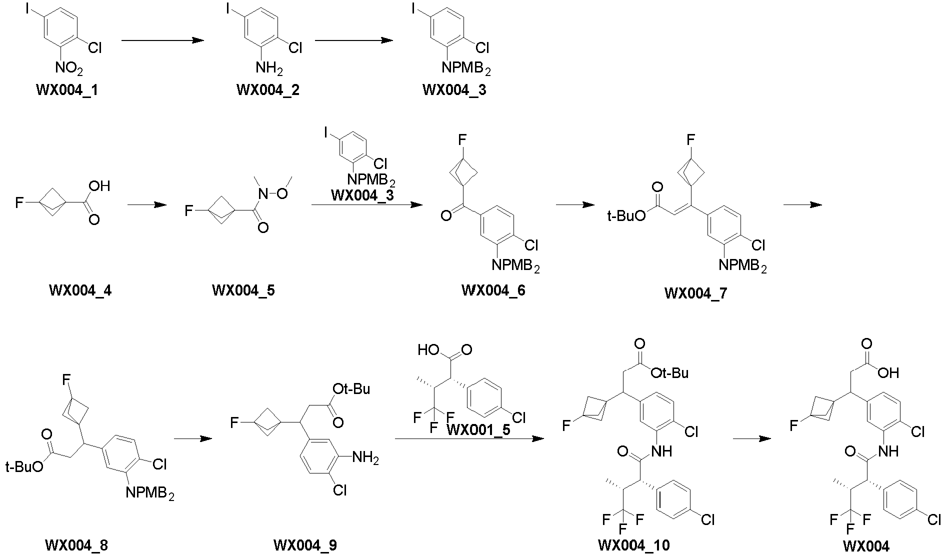

### Step 1: Synthesis of compound WX004_2

Compound **WX004_1** (7.8 g, 27.52 mmol) was dissolved in methanol (80 mL) at room temperature under nitrogen atmosphere. Iron powder (7.68 g, 137.59 mmol) was added thereto, followed by a dropwise addition of a solution of ammonium chloride (11.78 g, 220.14 mmol) in water (40 mL). The reaction system was stirred at 70°C for 12 hours. After the reaction was complete, the reaction mixture was filtered. The filter cake was washed with methanol (50 mL × 2). The filtrate was collected, and concentrated under reduced pressure to remove the solvent. The reaction mixture was diluted with water (50 mL) and then extracted with ethyl acetate (100 mL × 2). The organic phases were combined. The organic phase was washed with saturated brine (50 mL × 2), dried over anhydrous sodium sulfate, filtered, and then concentrated under reduced pressure to remove the solvent. The resulting residue was purified by column chromatography (eluent: petroleum ether/ethyl acetate = 1/0 to 5/1, v/v) to obtain compound **WX004_2**.

### Step 2: Synthesis of compound WX004_3

To N,N-dimethylformamide (50 mL) was added sodium hydride (1.10 g, 27.42 mmol, purity: 60%) in batches at 0°C under nitrogen atmosphere. A solution of compound **WX004_2** (2.78 g, 10.97 mmol) in N,N-dimethylformamide (30 mL) was then slowly added dropwise to the reaction system. After the reaction system was stirred at 0°C for 0.5 hours, 4-methoxybenzylchloride (4.09 g, 26.10 mmol, 3.55 mL) was added dropwise thereto. Following the addition, the reaction system was warmed to 25°C and stirred for 12 hours. After the reaction was complete, the reaction system was slowly pouring into water (50 mL) to quench and diluted with ethyl acetate (100 mL), and the phases were separated. The organic phase was collected, and the aqueous phase was extracted with ethyl acetate (50 mL × 3). The organic phases were then combined. The organic phase was washed with saturated brine (50 mL × 2), dried over anhydrous sodium sulfate, and filtered. The filtrate was concentrated under reduced pressure to remove the solvent. The resulting residue was purified by column chromatography (eluent: petroleum ether/ethyl acetate = 1/0 to 5/1, v/v) to obtain compound **WX004_3**. ¹H NMR (400 MHz, CDCl₃) δ: 7.36-7.23 (m, 6H), 7.18 (d, *J*=8.4 Hz, 1H), 6.93 (d, *J*=8.5 Hz, 4H), 4.18 (s, 4H), 3.89 (s, 6H).

### Step 3: Synthesis of compound WX004_5

**WX004_4** (500 mg, 3.84 mmol) and methoxymethylamine hydrochloride (412.32 mg, 4.23 mmol) were dissolved in ethyl acetate (10 mL) at room temperature under nitrogen atmosphere. N-Methylmorpholine (1.17 g, 11.53 mmol, 1.27 mL) was added dropwise thereto, followed by a dropwise addition of 1-propylphosphonic acid cyclic anhydride (3.67 g, 5.76 mmol, 3.43 mL, 50% solution in ethyl acetate). The reaction mixture was stirred at 20°C for 12 hours. After the reaction was complete, the reaction mixture was poured into water (20 mL) to quench the reaction. The phases were separated, and the organic phase was collected. The aqueous phase was extracted with ethyl acetate (10 mL × 2), and the organic phases were combined. The organic phase was washed with saturated brine (30 mL), dried over anhydrous sodium sulfate, and filtered. The filtrate was concentrated under reduced pressure to remove the solvent. The resulting residue was purified by column chromatography (eluent: petroleum ether/ethyl acetate = 19/1 to 3/1, v/v) to obtain compound **WX004_5**. ¹H NMR (400 MHz, CDCl₃) δ: 3.67 (s, 3H), 3.20 (s, 3H), 2.41 (d, *J*=2.5 Hz, 6H).

### Step 4: Synthesis of compound WX004_6

Compound **WX004_3** (1.57 g, 3.18 mmol) was dissolved in tetrahydrofuran (17 mL) at room temperature under nitrogen atmosphere. The reaction mixture was cooled to -70°C, and a 2 M solution of isopropylmagnesium chloride in tetrahydrofuran (1.59 mL, 3.18 mmol) was added dropwise thereto. After the addition was complete, the reaction mixture was warmed to -40°C and stirred for 0.5 hours. A solution of compound **WX004_5** (220 mg, 1.27 mmol) in tetrahydrofuran (5 mL) was then added dropwise thereto. After the addition was complete, the reaction mixture was warmed to 20°C and stirred for 12 hours. After the reaction was complete, the reaction mixture was slowly poured into saturated ammonium chloride solution (50 mL) to quench the reaction. The phases were separated, and the organic phase was collected. The aqueous phase was extracted with ethyl acetate (20 mL × 2), and the organic phases were combined. The organic phase was dried over anhydrous sodium sulfate, filtered, and the filtrate was concentrated under reduced pressure to remove the solvent. The resulting crude product was purified by column chromatography (eluent: petroleum ether/ethyl acetate = 1/0 to 24/1) to obtain compound **WX004_6**. ¹H NMR (400 MHz, CDCl₃) δ: 7.52-7.45 (m, 2H), 7.41 (s, 1H), 7.20 (d, *J*=8.4 Hz, 4H), 6.82 (d, *J*=8.6 Hz, 4H), 4.17 (s, 4H), 3.78 (s, 6H), 2.44 (d, *J*=2.2 Hz, 6H).

### Step 5: Synthesis of compound WX004_7

tert-Butyl diethylphosphonoacetate (235.39 mg, 933.18 µmol) was dissolved in tetrahydrofuran (5 mL) at room temperature under nitrogen atmosphere. A 1 M solution of potassium tert-butoxide in tetrahydrofuran (1.03 mL, 1.03 mmol) was added dropwise thereto at 0°C. After the addition was complete, the reaction mixture was stirred at 0°C for 0.5 hours. A solution of **WX004_6** (447.9 mg, 933.18 µmol) in tetrahydrofuran (3 mL) was then added dropwise thereto. After the addition was complete, the reaction mixture was stirred at 20°C for 12 hours. After the reaction was complete, the reaction mixture was poured into ice water (10 mL) to quench the reaction, extracted with ethyl acetate (15 mL × 3), and the organic phases were combined. The combined organic phase was washed with saturated brine (10 mL), dried over anhydrous sodium sulfate, and filtered. The filtrate was then concentrated under reduced pressure to remove the solvent. The resulting crude product was purified by column chromatography (eluent: petroleum ether/ethyl acetate = 99/1 to 97/3) to obtain target compound **WX004_7**. ¹H NMR (400 MHz, CDCl₃) δ: 7.37 (d, *J*=8.0 Hz, 1H), 7.21 (d, *J*=8.6 Hz, 4H), 6.85-6.80 (m, 4H), 6.62 (dd, *J*=2.0, 8.0 Hz, 1H), 6.51 (d, *J*=1.6 Hz, 1H), 5.74 (s, 1H), 4.13 (s, 4H), 3.78 (s, 6H), 1.87 (d, *J*=2.4 Hz, 6H), 1.22 (s, 9H)

### Step 6: Synthesis of compound WX004_8

Platinum on carbon (100 mg, purity: 5%) was added to a dry reaction flask under argon atmosphere and wetted with tetrahydrofuran (2 mL), followed by the addition of a solution of compound **WX004_7** (300 mg, 518.93 µmol) in tetrahydrofuran (2 mL). The reaction system was replaced with hydrogen three times and then reacted at 20°C under hydrogen (103kPa (15 psi)) for 12 hours. After the reaction was complete, the reaction mixture was filtered through diatomite. The filter cake was washed with tetrahydrofuran (10 mL × 3). The filtrates were combined, and then concentrated under reduced pressure to remove the solvent. The resulting crude product was purified by column chromatography (eluent: petroleum ether/ethyl acetate = 99/1 to 24/1, v/v) to obtain target compound **WX004_8**.

### Step 7: Synthesis of compound WX004_9

Compound **WX004_8** (78 mg, 134.45 µmol) was dissolved in a mixed solvent of dichloromethane (3.5 mL) and water (0.6 mL) at room temperature under nitrogen atmosphere. The reaction mixture was cooled to 0°C, and dichlorodicyanobenzoquinone (92.28 mg, 406.54 µmol) was added thereto in batches. The reaction mixture was then stirred at 20°C for 2 hours. After the reaction was complete, the reaction mixture was added with saturated sodium bicarbonate aqueous solution (20 mL) and stirred for 0.5 hours. The phases were then separated, and the organic phase was collected. The aqueous phase was extracted with dichloromethane (10 mL × 2), and the organic phases were combined. The organic phase was dried over anhydrous sodium sulfate, filtered, and the filtrate was concentrated under reduced pressure to remove the solvent. The resulting crude product was purified by column chromatography (eluent: petroleum ether/ethyl acetate = 1/0 to 19/1, v/v) to obtain target compound **WX004_9.**

### Step 8: Synthesis of compound WX004_10

Compound **WX001_5** (55.71 mg, 208.93 µmol) was dissolved in dichloromethane (1 mL) at room temperature under nitrogen atmosphere. The reaction mixture was cooled to 0°C, and oxalyl chloride (45.72 µL, 522.32 µmol) was added dropwise thereto, followed by the addition of a drop of N,N-dimethylformamide. The reaction mixture was stirred at 20°C for 1 hour, and concentrated under reduced pressure to remove the solvent. Dichloromethane (1 mL) was added to dissolve the residue, and the reaction mixture was again concentrated under reduced pressure to obtain a crude product. The crude product was dissolved in dichloromethane (1 mL), and added dropwise to a solution of compound **WX004_9** (71 mg, 208.93 µmol) and N,N-diisopropylethylamine (67.51 mg, 522.32 µmol) in dichloromethane (1 mL) at 0°C. The reaction mixture was then stirred at 20°C for 12 hours. After the reaction was complete, the reaction mixture was poured into water (10 mL) to quench the reaction, extracted with dichloromethane (5 mL × 3), and the organic phases were combined. The organic phase was washed with saturated brine (20 mL), dried over anhydrous sodium sulfate, filtered, and the filtrate was concentrated under reduced pressure to remove the solvent. The resulting crude product was purified by column chromatography (eluent: petroleum ether/ethyl acetate = 46/1 to 17/3, v/v) to obtain target compound **WX004_10**. ¹H NMR (400 MHz, CDCl₃) δ: 8.11 (d, *J*=1.6 Hz, 1H), 7.59 (d, *J*=5.6 Hz, 1H), 7.41-7.31 (m, 4H), 7.25 (dd, *J*=2.0, 8.4 Hz, 1H), 6.80 (d, *J*=8.0 Hz, 1H), 3.69-3.65 (m, 1H), 3.47-3.43 (m, 1H), 3.41-3.33 (m, 1H), 2.67-2.49 (m, 2H), 1.90-1.82 (m, 6H), 1.33 (d, *J*=2.4 Hz, 9H), 0.96 (d, *J*=7.2 Hz, 3H).

### Step 9: Synthesis of compound WX004

Compound **WX004_10** (100 mg, 169.94 µmol) was dissolved in ethyl acetate (0.5 mL) at room temperature under nitrogen atmosphere. A solution of hydrochloric acid in ethyl acetate (4 M, 2 mL) was added thereto, and the reaction system was stirred at 25°C for 1 hour. After the reaction was complete, the reaction mixture was directly concentrated under reduced pressure to remove the solvent. The resulting residue was purified by preparative HPLC (mobile phase: acetonitrile/water; acidic system: 0.04% HCl) to obtain target compound WX004. MS-ESI m/z: 531.9 [M+H]⁺. ¹H NMR (400 MHz, DMSO_*d*₆) δ: 12.16 (s, 1H), 9.84 (d, *J*=3.1 Hz, 1H), 7.52-7.43 (m, 4H), 7.39-7.32 (m, 2H), 7.04-6.92 (m, 1H), 4.13 (d, *J*=10.8 Hz, 1H), 3.41-3.34 (m, 2H), 2.65-2.57 (m, 1H), 2.55-2.54 (m, 1H), 1.83-1.73 (m, 6H), 0.80 (d, *J*=6.8 Hz, 3H).

### Example 5

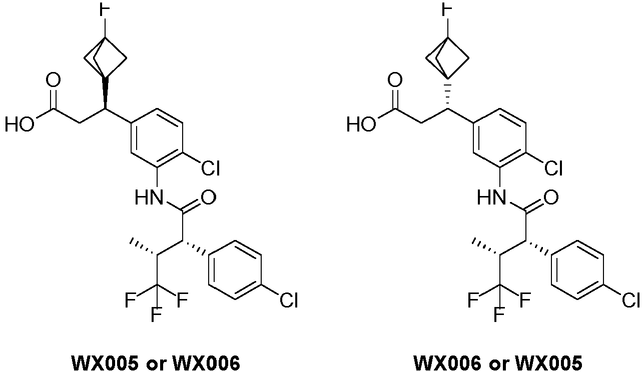

### Step 1: Synthesis of compounds WX005 and WX006

Compound **WX004** was initially separated by chiral column chromatography (column type: DAICEL CHIRALPAK IC (250 mm × 30 mm, 10 µm); mobile phase: A (CO₂) and B (isopropanol, containing 0.1% diisopropylamine); gradient: B% = 33% to 33%, 5 minutes) and then purified by preparative HPLC (mobile phase: acetonitrile/water; acidic system: 0.04% HCl) to obtain compounds **WX005** and **WX006,** respectively.

SFC analysis method: column type: Chiralpak IC-3 (100 × 4.6 mm I.D., 3µm); mobile phase: A: CO₂, B: [IPA (containing 0.1% IPAm)], gradient: B%: 5% to 40%, 3 minutes.

**WX005** (retention time: 1.874 min): MS-ESI m/z: 532. 1[M+H]⁺. ee value: 100%. ¹H NMR (400 MHz, DMSO_*d*₆) δ: 12.16 (s, 1H), 9.85 (s, 1H), 7.50-7.42 (m, 4H), 7.36 (d, *J*=8.4 Hz, 1H), 7.34 (d, *J*=2.0 Hz, 1H), 6.97 (dd, *J*=2.0, 8.4 Hz, 1H), 4.13 (d, *J*=10.8 Hz, 1H), 3.43-3.39 (m, 2H), 2.64-2.58 (m, 1H), 2.54-2.52 (m, 1H), 1.86-1.70 (m, 6H), 0.80 (d, *J*=6.8 Hz, 3H).

**WX006** (retention time: 2.003 min): MS-ESI m/z: 532.1[M+H]⁺. ee value: 92.44%. ¹H NMR (400 MHz, DMSO_*d*₆) δ: 12.14 (s, 1H), 9.84 (s, 1H), 7.53-7.41 (m, 4H), 7.40-7.30 (m, 2H), 6.97 (d, *J*=8.0 Hz, 1H), 4.13 (d, *J*=10.8 Hz, 1H), 3.39-3.36 (m, 2H), 2.63-2.58 (m, 1H), 2.54-2.53 (m, 1H), 1.86-1.71 (m, 6H), 0.80 (d, *J*=6.8 Hz, 3H).

### Example 7

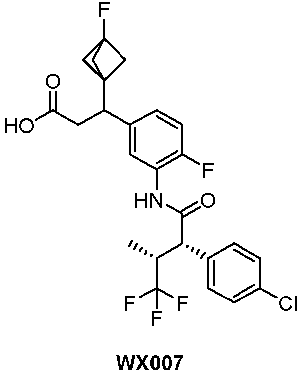

### Synthetic route:

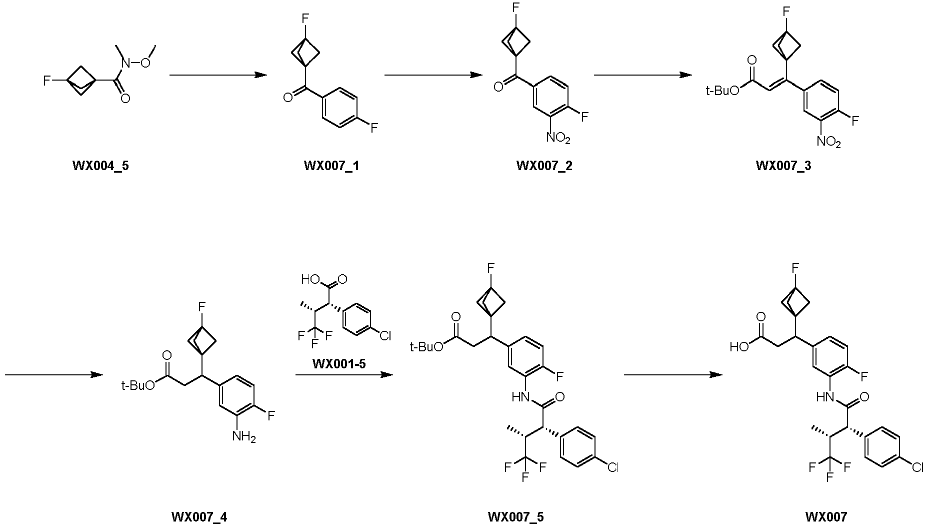

### Step 1: Synthesis of compound WX007_1

To a dry reaction flask was added 4-fluorophenylmagnesium bromide (13.86 mL, 4.62 mmol, 1 M solution in tetrahydrofuran) at room temperature under nitrogen atmosphere. The reaction mixture was cooled to -30°C under nitrogen atmosphere, and a solution of compound **WX004_5** (0.8 g, 4.62 mmol) in tetrahydrofuran (10 mL) was added dropwise thereto. After the addition was complete, the reaction mixture was warmed to 20°C and stirred for 12 hours. After the reaction was complete, the reaction mixture was poured into saturated ammonium chloride aqueous solution (20 mL) to quench the reaction, and then extracted with ethyl acetate

(10 mL × 2). The organic phases were combined, dried over anhydrous sodium sulfate, and filtered. The filtrate was concentrated under reduced pressure to remove the solvent. The resulting residue was purified by column chromatography (eluent, petroleum ether/ethyl acetate = 1/0 to 97/3, v/v) to obtain target compound **WX007_1.**

### Step 2: Synthesis of compound WX007_2

Fuming nitric acid (6.02 g, 95.54 mmol, 4.30 mL, purity: 95%) was placed in a dry reaction flask at room temperature under nitrogen atmosphere. The reaction mixture was then cooled to -10°C. Compound **WX007_1** (390 mg, 1.87 mmol) was slowly added thereto in batches, and the reaction mixture was stirred at 0°C for 1 hour. After the reaction was complete, the reaction mixture was slowly poured into ice water (10 mL) to quench the reaction, and then extracted with ethyl acetate (15 mL × 2). The organic phases were combined, washed with saturated sodium bicarbonate aqueous solution (10 mL), dried over anhydrous sodium sulfate, and filtered. The filtrate was concentrated under reduced pressure to remove the solvent. The resulting residue was purified by column chromatography (eluent, petroleum ether/ethyl acetate = 1/0 to 97/3, v/v) to obtain target compound **WX007_2**.

### Step 3: Synthesis of compound WX007_3

tert-Butyl diethylphosphonoacetate (318.79 mg, 1.26 mmol) was dissolved in tetrahydrofuran (3 mL) at room temperature under nitrogen atmosphere. The reaction mixture was cooled to 0°C under nitrogen atmosphere, and a 1 M solution of potassium tert-butoxide in tetrahydrofuran (1.39 mL, 1.39 mmol) was added dropwise thereto. After the addition was complete, the reaction mixture was stirred for 0.5 hours. A solution of compound **WX007_2** (320 mg, 1.26 mmol) in tetrahydrofuran (3 mL) was then added dropwise thereto, and the reaction mixture was stirred at 20°C for 12 hours. After the reaction was complete, the reaction mixture was poured into water (20 mL), and extracted with ethyl acetate (10 mL × 3). The organic phase was washed with saturated brine (30 mL), dried over anhydrous sodium sulfate, filtered, and the filtrate was concentrated under reduced pressure to remove the solvent. The resulting residue was purified by column chromatography (eluent, petroleum ether/ethyl acetate = 1/0 to 97/3, v/v) to obtain target compound **WX007_3**. ¹H NMR (400 MHz, CDCl₃) δ: 7.76 (dd, *J*=2.0, 7.0 Hz, 1H), 7.34-7.30 (m, 1H), 7.27-7.22 (m, 1H), 5.91 (s, 1H), 2.14 (d, *J*=2.4 Hz, 6H), 1.27 (s, 9H).

### Step 4: Synthesis of compound WX007_4

Platinum on carbon (150.00 mg, purity: 5%) was placed in a reaction flask under argon atmosphere and wetted with tetrahydrofuran (3 mL). A solution of compound **WX007_3** (300 mg, 853.86 µmol) in tetrahydrofuran (2 mL) was then added thereto. The reaction mixture was replaced with hydrogen three times, and reacted at 20°C under hydrogen atmosphere of 103 kPa (15 psi) for 12 hours. After the reaction was complete, the reaction mixture was filtered through diatomite. The filter cake was washed with tetrahydrofuran (5 mL). The filtrates were combined and concentrated under reduced pressure to remove the solvent. Thus target compound **WX007_4** was obtained, and the crude product was directly used in the next step.

### Step 5: Synthesis of compound WX007_5

Compound **WX001_5** (340 mg, 1.28 mmol) was dissolved in dichloromethane (4 mL) at room temperature under nitrogen atmosphere. The reaction mixture was cooled to 0°C, and oxalyl chloride (404.62 mg, 3.19 mmol, 279.05 µL) was added dropwise thereto. A drop of N,N-dimethylformamide was then added thereto, and the reaction mixture was stirred at 20°C for 1 hour. After the reaction was complete, the reaction mixture was directly concentrated under reduced pressure to remove the solvent. The residue was dissolved in dichloromethane (1 mL). To a solution of compound **WX007_4** (210 mg, 649.40 µmol) and N,N-diisopropylethylamine (209.82 mg, 1.62 mmol, 282.78 µL) in dichloromethane (2 mL) was added dropwise the prepared acyl chloride at 0°C. The reaction mixture was then stirred at 20°C for 12 hours. After the reaction was complete, the reaction mixture was concentrated under reduced pressure to remove the solvent. The resulting residue was purified by column chromatography (eluent, petroleum ether/ethyl acetate = 49/1 to 17/3, v/v) to obtain target compound **WX007_5.**

### Step 6: Synthesis of compound WX007

Compound **WX007_5** (70 mg, 122.38 µmol) was dissolved in a solution of hydrochloric acid in ethyl acetate (4 M, 2 mL) at room temperature under nitrogen atmosphere. The reaction system was stirred at 20°C for 2 hours. After the reaction was complete, the reaction mixture was directly concentrated under reduced pressure to remove the solvent. The residue was purified by preparative HPLC (mobile phase: acetonitrile/water; acidic system: 0.04% HCl) to obtain target compound **WX007**. MS-ESI m/z: 515.9 [M+H]⁺. ¹H NMR (400 MHz, DMSO_*d*₆) δ: 12.12 (s, 1H), 10.06 (s, 1H), 7.58-7.61 (m, 1H), 7.49-7.42 (m, 4H), 7.18-7.10 (m, 1H), 6.96-6.88 (m, 1H), 4.11 (dd, *J*=2.6, 10.6 Hz, 1H), 3.37 (s, 2H), 2.63-2.57 (m, 1H), 2.52-2.51 (m, 1H), 1.82-1.72 (m, 6H), 0.79 (d, *J*=7.0 Hz, 3H).

### Example 8

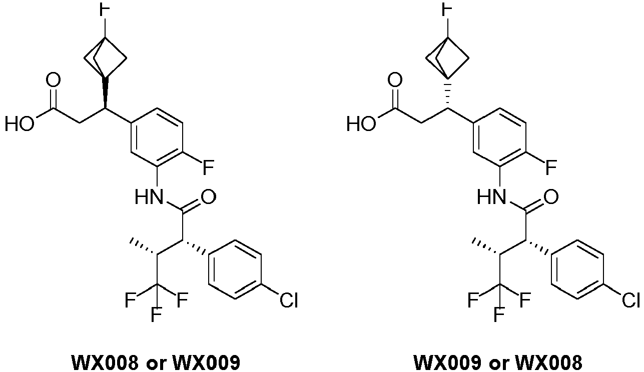

### Step 1: Synthesis of compounds WX008 and WX009

Compound **WX007** was initially separated by chiral column chromatography (column type: DAICEL CHIRALPAK IC (250 mm × 30 mm, 10 µm); mobile phase: A (CO₂) and B (isopropanol containing 0.1% ammonia); gradient: B% = 20% to 20%, 4 minutes) and then purified by preparative HPLC (mobile phase: acetonitrile/water; acidic system: 0.04% HCl) to obtain compounds **WX008** and **WX009,** respectively.

SFC analysis method: column type: Chiralpak IC-3, 50 × 4.6 mm I.D., 3 µm; mobile phase: A: CO₂, B: [IPA (containing 0.1% IPAm)], gradient: B%: 5% to 50%, 3 minutes.

**WX008** (retention time: 0.966 min): MS-ESI m/z: 515.9 [M+H]⁺. ee value: 100%. ¹H NMR (400 MHz, DMSO_*d*₆) δ: 12.13 (s, 1H), 10.07 (s, 1H), 7.60 (dd, *J*=2.2, 7.4 Hz, 1H), 7.50-7.43 (m, 4H), 7.15 (dd, *J*=8.4, 10.8 Hz, 1H), 6.95-6.89 (m, 1H), 4.12 (d, *J*=10.8 Hz, 1H), 3.42-3.36 (m, 2H), 2.64-2.57 (m, 1H), 2.55-2.52 (m, 1H), 1.85-1.72 (m, 6H), 0.80 (d, *J*=7.0 Hz, 3H).

**WX009** (retention time: 1.021 min): MS-ESI m/z: 516.2 [M+H]⁺. ee value: 90.3%. ¹H NMR (400 MHz, DMSO_*d*₆) δ: 12.13 (s, 1H), 10.07 (s, 1H), 7.61 (dd, *J*=2.2, 7.4 Hz, 1H), 7.49-7.44 (m, 4H), 7.15 (dd, *J*=8.4, 10.8 Hz, 1H), 6.94-6.91 (m, 1H), 4.12 (d, *J*=10.8 Hz, 1H), 3.42-3.38 (m, 2H), 2.65-2.57 (m, 1H), 2.64-2.57 (m, 1H), 1.92-1.60 (m, 6H), 0.79 (d, *J*=7.0 Hz, 3H).

### Example 10

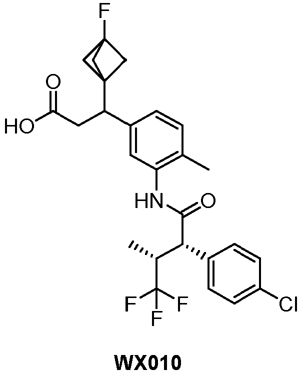

### Synthetic route:

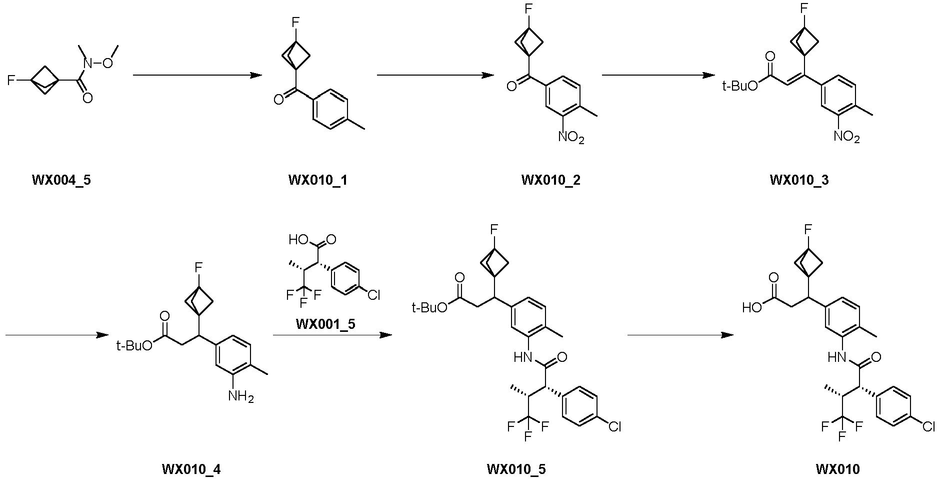

### Step 1: Synthesis of compound WX010_1

To a dry reaction flask was added 4-methylphenylmagnesium chloride (2.89 mL, 5.77 mmol, 2 M solution in tetrahydrofuran) at room temperature under nitrogen atmosphere. The reaction mixture was cooled to -30°C under nitrogen atmosphere, and a solution of compound **WX004_5** (0.4 g, 2.31 mmol) in tetrahydrofuran (5 mL) was added dropwise thereto. After the addition was complete, the reaction mixture was warmed to 20°C and stirred for 12 hours. After the reaction was complete, the reaction mixture was poured into saturated ammonium chloride aqueous solution (20 mL) to quench the reaction, and then extracted with ethyl acetate (10 mL × 2). The organic phases were combined, dried over anhydrous sodium sulfate, and filtered. The filtrate was concentrated under reduced pressure to remove the solvent. The resulting residue was purified by column chromatography (eluent, petroleum ether/ethyl acetate = 1/0 to 97/3, v/v) to obtain target compound **WX010_1.**

### Step 2: Synthesis of compound WX010_2

Fuming nitric acid (1.12 g, 17.77 mmol, 0.80 mL, purity: 95%) was placed in a dry reaction flask at room temperature under nitrogen atmosphere. The reaction mixture was then cooled to -10°C. Compound **WX010_1** (120 mg, 587.55 µmol) was slowly added thereto in batches, and the reaction mixture was stirred at 0°C for 1 hour. After the reaction was complete, the reaction mixture was slowly poured into ice water (10 mL) to quench the reaction, and then extracted with ethyl acetate (5 mL × 2). The organic phases were combined, washed with saturated sodium bicarbonate aqueous solution (10 mL), dried over anhydrous sodium sulfate, and filtered. The filtrate was concentrated under reduced pressure to remove the solvent. The residue was purified by column chromatography (eluent, petroleum ether/ethyl acetate = 1/0 to 97/3, v/v) to obtain target compound **WX010_2**. ¹H NMR (400 MHz, CDCl₃) δ: 8.53 (d, *J*=1.8 Hz, 1H), 8.06 (dd, *J*=1.8, 8.0 Hz, 1H), 7.48 (d, *J*=8.0 Hz, 1H), 2.69 (s, 3H), 2.63 (d, *J*=2.4 Hz, 6H).

### Step 3: Synthesis of compound WX010_3

tert-Butyl diethylphosphonoacetate (86.03 mg, 341.04 µmol) was dissolved in tetrahydrofuran (1 mL) at room temperature under nitrogen atmosphere. The reaction mixture was cooled to 0°C under nitrogen atmosphere, and a 1 M solution of potassium tert-butoxide in tetrahydrofuran (375.14 µL, 375.14 µmol) was added dropwise thereto. After the addition was complete, the reaction mixture was stirred for 0.5 hours. A solution of compound **WX010_2** (85 mg, 341.04 µmol) in tetrahydrofuran (1 mL) was then added dropwise thereto, and the reaction mixture was stirred at 20°C for 12 hours. After the reaction was complete, the reaction mixture was poured into water (20 mL), and extracted with ethyl acetate (10 mL × 3). The organic phases were combined, washed with saturated brine (30 mL), dried over anhydrous sodium sulfate, filtered, and the filtrate was concentrated under reduced pressure to remove the solvent. The residue was purified by column chromatography (eluent, petroleum ether/ethyl acetate = 1/0 to 97/3, v/v) to obtain target compound **WX010_3**.

### Step 4: Synthesis of compound WX010_4

Platinum on carbon (40.00 mg, purity: 5%) was placed in a reaction flask under argon atmosphere and wetted with tetrahydrofuran (3 mL). A solution of compound **WX010_3** (80 mg, 230.30 µmol) in tetrahydrofuran (1 mL) was then added thereto. The reaction mixture was replaced with hydrogen three times, and stirred at 20°C under hydrogen atmosphere of 103 kPa (15 psi) for 12 hours. After the reaction was complete, the reaction mixture was filtered through diatomite. The filter cake was washed with tetrahydrofuran (5 mL). The filtrates were combined and concentrated under reduced pressure to remove the solvent. Thus compound **WX010_4** was obtained, and the crude product was directly used in the next step.

### Step 5: Synthesis of compound WX010_5

Compound **WX001_5** (70.12 mg, 262.98 µmol) was dissolved in dichloromethane (4 mL) at room temperature under nitrogen atmosphere, and the reaction mixture was cooled to 0°C. To the reaction mixture was added dropwise oxalyl chloride (69.54 mg, 547.88 µmol, 47.96 µL), followed by the addition of a drop of N,N-dimethylformamide. The reaction mixture was stirred at 20°C for 1 hour. After the reaction was complete, the reaction mixture was directly concentrated under reduced pressure to remove the solvent. The residue was dissolved in dichloromethane (1 mL). To a solution of compound **WX010_4** (70 mg, 219.15 µmol) and N,N-diisopropylethylamine (70.81 mg, 547.88 µmol, 95.43 µL) in dichloromethane (2 mL) was added dropwise a prepared solution of acyl chloride in dichloromethane at 0°C. The reaction mixture was then stirred at 20°C for 12 hours. After the reaction was complete, the reaction mixture was concentrated under reduced pressure to remove the solvent. The resulting residue was purified by column chromatography (eluent, petroleum ether/ethyl acetate = 49/1 to 17/3, v/v) to obtain target compound **WX010_5**. ¹H NMR (400 MHz, CDCl₃) δ: 7.45 (s, 1H), 7.42-7.28 (m, 4H), 7.05 (d, *J*=7.2 Hz, 1H), 6.90 (s, 1H), 6.82 (d, *J*=7.6 Hz, 1H), 3.65 (dd, *J*=4.8, 8.4 Hz, 1H), 3.42 (t, *J*=7.8 Hz, 2H), 2.63-2.49 (m, 2H), 2.03 (d, *J*=8.2 Hz, 3H), 1.84 (s, 6H), 1.33 (s, 9H), 0.95 (d, *J*=7.2 Hz, 3H).

### Step 6: Synthesis of compound WX010

Compound **WX010_5** (118 mg, 207.73 µmol) was dissolved in a solution of hydrochloric acid in ethyl acetate (4 M, 2.95 mL) at room temperature under nitrogen atmosphere. The reaction system was stirred at 20°C for 2 hours. After the reaction was complete, the reaction mixture was directly concentrated under reduced pressure to remove the solvent. The residue was purified by preparative HPLC (mobile phase: acetonitrile/water; acidic system: 0.04% HCl) to obtain target compound **WX010**. MS-ESI m/z: 512.0 [M+H]⁺. ¹H NMR (400 MHz, DMSO_*d*₆) δ: 12.10 (s, 1H), 9.60 (d, *J*=4.0 Hz, 1H), 7.46 (d, *J*=1.3 Hz, 4H), 7.11-7.04 (m, 1H), 7.04-6.96 (m, 1H), 6.89-6.82 (m, 1H), 3.97 (d, *J*=10.8 Hz, 1H), 3.45-3.35 (m, 2H), 2.62-2.55 (m, 1H), 2.52 (d, *J*=1.9 Hz, 1H), 1.96 (d, *J*=15.4 Hz, 3H), 1.82-1.72 (m, 6H), 0.81 (d, *J*=7.0 Hz, 3H).

### Example 11

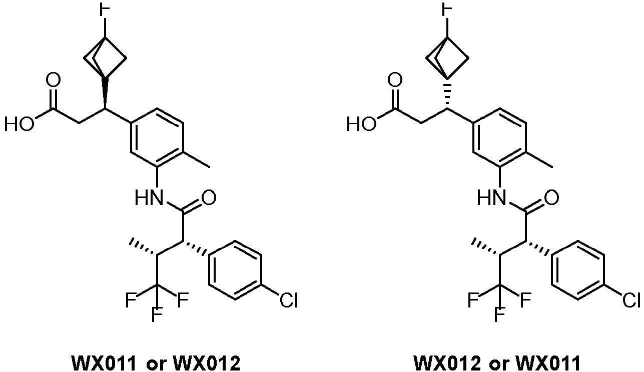

### Step 1: Synthesis of compounds WX011 and WX012

Compound **WX010** was initially separated by chiral column chromatography (column type: DAICEL CHIRALCEL OX (250 mm × 30 mm, 10 µm); mobile phase: A (CO₂) and B (isopropanol containing 0.1% ammonia); gradient: B% = 30% to 30%, 7 minutes) and then purified by preparative HPLC (mobile phase: acetonitrile/water; acidic system: 0.04% HCl) to obtain compounds **WX011** and **WX012,** respectively.

SFC analysis method: column type: Chiralcel OX-3, 50 × 4.6 mm I.D., 3µm; mobile phase: A: CO₂, B: [IPA (containing 0.1% IPAm)], gradient: B%: 5% to 50%, 3 minutes.

**WX011** (retention time: 1.119 min): MS-ESI m/z: 512.2 [M+H]⁺. ee value: 100%. ¹H NMR (400 MHz, DMSO_*d*₆) δ: 12.10 (s, 1H), 9.61 (s, 1H), 7.46 (s, 4H), 7.08 (d, *J*=8.0 Hz, 1H), 7.01 (d, *J*=1.6 Hz, 1H), 6.86 (dd, *J*=1.6, 7.8 Hz, 1H), 3.97 (d, *J*=10.6 Hz, 1H), 3.44-3.34 (m, 2H), 2.62-2.56 (m, 1H), 2.52 (d, *J*=1.9 Hz, 1H), 1.95 (s, 3H), 1.83-1.73 (m, 6H), 0.81 (d, *J*=7.0 Hz, 3H).

**WX012** (retention time: 1.208 min): MS-ESI m/z: 512.2 [M+H]⁺. ee value: 100%. ¹H NMR (400 MHz, DMSO_*d*₆) δ: 12.09 (s, 1H), 9.60 (s, 1H), 7.46 (s, 4H), 7.08 (d, *J*=8.0 Hz, 1H), 6.99 (s, 1H), 6.86 (d, *J*=7.8 Hz, 1H), 3.97 (d, *J*=10.8 Hz, 1H), 3.46-3.33 (m, 2H), 2.62-2.54 (m, 1H), 2.52 (s, 1H), 1.98 (s, 3H), 1.82-1.72 (m, 6H), 0.81 (d, *J*=7.0 Hz, 3H).

### Example 13

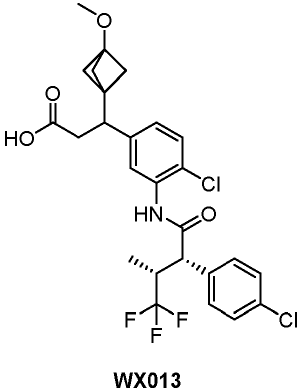

### Synthetic route:

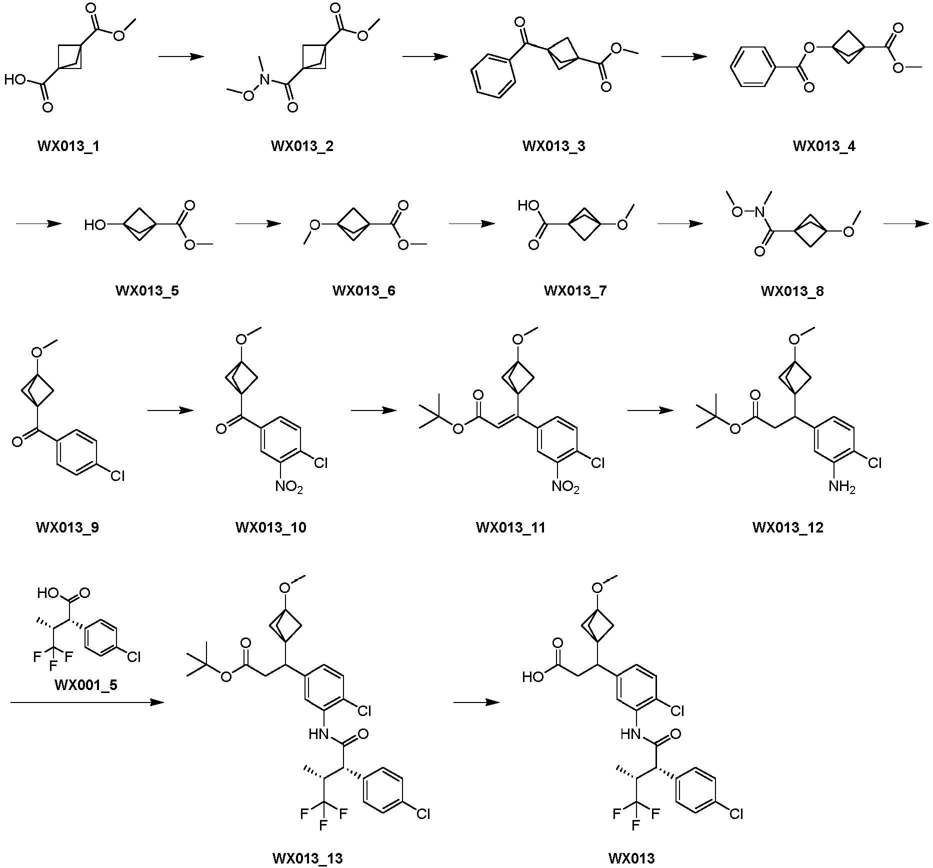

### Step 1: Synthesis of compound WX013_2

**WX013_1** (8.0 g, 47.01 mmol) and methoxymethylamine hydrochloride (5.04 g, 51.72 mmol) were dissolved in ethyl acetate (150 mL) at room temperature under nitrogen atmosphere. N-methylmorpholine (14.27 g, 141.04 mmol, 15.51 mL) was added dropwise thereto, followed by a dropwise addition of 1-propylphosphonic acid cyclic anhydride (44.88 g, 70.52 mmol, 41.94 mL, 50% solution in ethyl acetate). The reaction mixture was stirred at 20°C for 12 hours. After the reaction was complete, the reaction mixture was poured into water (100 mL) to quench the reaction. The phases were separated, and the organic phase was collected. The aqueous phase was extracted with ethyl acetate (200 mL × 2), and the organic phases were combined. The organic phase was washed with saturated brine (200 mL × 2), dried over anhydrous sodium sulfate, and filtered. The filtrate was concentrated under reduced pressure to remove the solvent. The resulting residue was purified by column chromatography (eluent: petroleum ether/ethyl acetate = 1/0 to 1/1, v/v) to obtain compound **WX013_2**. ¹H NMR (400 MHz, DMSO_*d*₆) δ: 3.65 (s, 3H), 3.61 (s, 3H), 3.08 (s, 3H), 2.26 (s, 6H).

### Step 2: Synthesis of compound WX013_3

**WX013_2** (2.7 g, 12.66 mmol) was dissolved in anhydrous tetrahydrofuran (40 mL) at room temperature under nitrogen atmosphere. The reaction mixture was cooled to 0°C under nitrogen atmosphere. A 3 M solution of phenylmagnesium bromide in tetrahydrofuran (6.33 mL, 18.99 mmol) was then slowly added dropwise thereto. After the addition was complete, the reaction mixture was stirred at 0°C for 0.5 hours, then warmed to 20°C, and stirred for another 12 hours. After the reaction was complete, the reaction mixture was slowly poured into saturated ammonium chloride solution (100 mL) to quench the reaction, extracted with ethyl acetate (50 mL × 2), and the organic phases were combined. The organic phase was washed with saturated brine (50 mL × 2), dried over anhydrous sodium sulfate, and filtered. The filtrate was concentrated under reduced pressure to remove the solvent. The resulting residue was purified by column chromatography (eluent: petroleum ether/ethyl acetate = 1/0 to 9/1, v/v) to obtain compound **WX013_3**. ¹H NMR (400 MHz, CDCl₃) δ: 8.00-7.96 (m, 2H), 7.61-7.55 (m, 1H), 7.51-7.43 (m, 2H), 3.73 (s, 3H), 2.56 (s, 6H).

### Step 3: Synthesis of compound WX013_4

**WX013_3** (2.1 g, 9.12 mmol) was dissolved in anhydrous dichloromethane (25 mL) at room temperature under nitrogen atmosphere. The reaction mixture was cooled to 0°C under nitrogen atmosphere. m-Chloroperoxybenzoic acid (2.78 g, 13.68 mmol, purity: 85%) was then slowly added thereto in batches. After the addition was complete, the reaction mixture was stirred at 20°C for 2 hours, then cooled to 0°C, and an additional amount of m-chloroperoxybenzoic acid (2.78 g, 13.68 mmol, purity: 85%) was added thereto. The reaction mixture was subsequently warmed to 20°C, and stirred for another 12 hours. After the reaction was complete, the reaction mixture was slowly poured into saturated sodium sulfite solution (50 mL) to quench the reaction, and then extracted with dichloromethane (20 mL × 2). The organic phases were combined, dried over anhydrous sodium sulfate, and filtered. The filtrate was concentrated under reduced pressure to remove the solvent. The resulting residue was purified by column chromatography (eluent: petroleum ether/ethyl acetate = 19/1 to 17/3, v/v) to obtain compound **WX013_4.**

### Step 4: Synthesis of compound WX013_5

**WX013_4** (1.0 g, 4.06 mmol) was dissolved in a solution of hydrochloric acid in methanol (4 M, 20.00 mL) at room temperature under nitrogen atmosphere. The reaction mixture was heated to 50°C, and stirred for 4 hours. After the reaction was complete, the reaction mixture was directly concentrated under reduced pressure to remove the solvent. The resulting residue was purified by column chromatography (eluent: petroleum ether/ethyl acetate = 19/1 to 7/3, v/v) to obtain compound **WX013_5**. ¹H NMR (400 MHz, CDCl₃) δ: 3.69 (s, 3H), 2.85 (s, 1H), 2.22 (s, 6H).

### Step 5: Synthesis of compound WX013_6

**WX013_5** (374 mg, 2.63 mmol) was dissolved in anhydrous dichloromethane (20 mL) at room temperature under nitrogen atmosphere. 1,8-Bis(dimethylamino)naphthalene (2.31 g, 10.79 mmol) and trimethyloxonium tetrafluoroborate (1.21 g, 8.16 mmol) were then added thereto respectively. After the addition was complete, the reaction mixture was stirred at 20°C for 2 hours. After the reaction was complete, the reaction mixture was filtered. The filtrate was sequentially washed with 2 M dilute hydrochloric acid (20 mL) and saturated brine (20 mL), dried over anhydrous sodium sulfate, filtered, and concentrated under reduced pressure to remove the solvent. The resulting residue was purified by column chromatography (eluent: petroleum ether/ethyl acetate = 1/0 to 9/1, v/v) to obtain compound **WX013_6**. ¹H NMR (400 MHz, CDCl₃) δ: 3.70 (s, 3H), 3.31 (s, 3H), 2.18 (s, 6H).

### Step 6: Synthesis of compound WX013_7

**WX013_6** (322 mg, 2.06 mmol) was dissolved in a mixed solution of tetrahydrofuran (6 mL), methanol (2 mL), and water (2 mL) at room temperature under nitrogen atmosphere. Lithium hydroxide monohydrate (173.04 mg, 4.12 mmol) was then added thereto. After the addition was complete, the reaction mixture was stirred at 20°C for 12 hours. After the reaction was complete, the reaction mixture was diluted with water (10 mL). The pH of the reaction mixture was adjusted to approximately 2 to 3 using 1 M dilute hydrochloric acid. The reaction mixture was diluted with ethyl acetate (10 mL), and the phases were separated. The organic phase was collected, and the aqueous phase was extracted with ethyl acetate (10 mL × 2). The organic phases were then combined. The organic phase was washed with saturated brine (20 mL), dried over anhydrous sodium sulfate, and filtered. The filtrate was concentrated under reduced pressure to remove the solvent, and compound **WX013_7** was obtained. The crude product was directly used in the next step.

### Step 7: Synthesis of compound WX013_8

**WX013_7** (265 mg, 1.86 mmol) and methoxymethylamine hydrochloride (218.21 mg, 2.24 mmol) were dissolved in ethyl acetate (3 mL) at room temperature under nitrogen atmosphere. N-methylmorpholine (754.23 mg, 7.46 mmol, 819.82 µL) was added dropwise thereto, followed by a dropwise addition of 1-propylphosphonic acid cyclic anhydride (1.78 g, 2.80 mmol, 1.66 mL, 50% solution in ethyl acetate). The reaction mixture was stirred at 20°C for 12 hours. After the reaction was complete, the reaction mixture was poured into water (10 mL) to quench the reaction. The phases were separated, and the organic phase was collected. The aqueous phase was extracted with ethyl acetate (10 mL × 2), and the organic phases were combined. The organic phase was washed with saturated brine (20 mL), dried over anhydrous sodium sulfate, and filtered. The filtrate was concentrated under reduced pressure to remove the solvent. The resulting residue was purified by column chromatography (eluent: petroleum ether/ethyl acetate = 19/1 to 17/3, v/v) to obtain compound **WX013_8**. ¹H NMR (400 MHz, CDCl₃) δ: 3.67 (s, 3H), 3.32 (s, 3H), 3.20 (s, 3H), 2.23 (s, 6H).

### Step 8: Synthesis of compound WX013_9

To a dry reaction flask was added 4-chlorophenylmagnesium bromide (4.54 mL, 4.54 mmol, 1 M solution in tetrahydrofuran) at room temperature under nitrogen atmosphere. The reaction mixture was cooled to -30°C under nitrogen atmosphere, and a solution of compound **WX013_8** (280 mg, 1.51 mmol) in tetrahydrofuran (5 mL) was added dropwise thereto. After the addition was complete, the reaction mixture was warmed to 20°C and stirred for 2 hours. After the reaction was complete, the reaction mixture was poured into saturated ammonium chloride aqueous solution (10 mL) to quench the reaction, and then extracted with ethyl acetate (10 mL × 2). The organic phases were combined, dried over anhydrous sodium sulfate, and filtered. The filtrate was concentrated under reduced pressure to remove the solvent. The residue was purified by column chromatography (eluent, petroleum ether/ethyl acetate = 1/0 to 19/1, v/v) to obtain target compound **WX013_9**.

### Step 9: Synthesis of compound WX013_10

Fuming nitric acid (3 g, 47.61 mmol, 2.14 mL, purity: 95%) was placed in a dry reaction flask at room temperature under nitrogen atmosphere. The reaction mixture was then cooled to -10°C. Compound **WX013_9** (160 mg, 675.98 µmol) was slowly added thereto in batches, and the reaction mixture was stirred at 0°C for 2 hours. After the reaction was complete, the reaction mixture was slowly poured into ice water (10 mL) to quench the reaction, and then extracted with ethyl acetate (5 mL × 2). The organic phases were combined, washed with saturated brine (10 mL), dried over anhydrous sodium sulfate, and filtered. The filtrate was concentrated under reduced pressure to remove the solvent. The residue was purified by column chromatography (eluent, petroleum ether/ethyl acetate = 1/0 to 9/1, v/v) to obtain target compound **WX013_10**. ¹H NMR (400 MHz, CDCl₃) δ: 8.45 (d, J=2.0 Hz, 1H), 8.09 (dd, *J*=2.0, 8.4 Hz, 1H), 7.67 (d, J=8.4 Hz, 1H), 3.37 (s, 3H), 2.44 (s, 6H).

### Step 10: Synthesis of compound WX013_11

tert-Butyl diethylphosphonoacetate (148.65 mg, 589.30 µmol) was dissolved in tetrahydrofuran (2 mL) at room temperature under nitrogen atmosphere. The reaction mixture was cooled to 0°C under nitrogen atmosphere, and a 1 M solution of potassium tert-butoxide in tetrahydrofuran (648.23 µL, 648.23 µmol) was added dropwise thereto. After the addition was complete, the reaction mixture was stirred for 0.5 hours. A solution of compound **WX013_10** (166 mg, 589.30 µmol) in tetrahydrofuran (1 mL) was then added thereto, and the reaction mixture was stirred at 20°C for 2 hours. After the reaction was complete, the reaction mixture was poured into water (10 mL), and extracted with ethyl acetate (10 mL × 3). The organic phase was washed with saturated brine (30 mL), dried over anhydrous sodium sulfate, filtered, and the filtrate was concentrated under reduced pressure to remove the solvent. The residue was purified by column chromatography (eluent, petroleum ether/ethyl acetate = 1/0 to 20/1, v/v) to obtain target compound **WX013_11**. ¹H NMR (400 MHz, CDCl₃) δ: 7.61 (d, *J*=2.0 Hz, 1H), 7.54 (d, *J*=8.3 Hz, 1H), 7.25-7.21 (m, 1H), 5.91 (s, 1H), 3.29 (s, 3H), 1.97 (s, 6H), 1.28 (s, 9H).

### Step 11: Synthesis of compound WX013_12

Platinum on carbon (80.00 mg, purity: 5%) was placed in a reaction flask under argon atmosphere and wetted with tetrahydrofuran (1.5 mL). A solution of compound **WX013_11** (160 mg, 421.24 µmol) in tetrahydrofuran (1.5 mL) was then added thereto. The reaction mixture was replaced with hydrogen three times, and stirred at 20°C under hydrogen atmosphere of 103 kPa (15 psi) for 16 hours. After the reaction was complete, the reaction mixture was filtered through diatomite. The filter cake was washed with tetrahydrofuran (5 mL). The filtrates were combined and concentrated under reduced pressure to remove the solvent. The residue was purified by column chromatography (eluent, petroleum ether/ethyl acetate = 1/0 to 9/1, v/v) to obtain target compound **WX013_12.**

### Step 12: Synthesis of compound WX013_13

Compound **WX001_5** (97.76 mg, 366.62 µmol) was dissolved in dichloromethane (1 mL) at room temperature under nitrogen atmosphere, and the reaction mixture was cooled to 0°C. To the reaction mixture was added dropwise oxalyl chloride (77.56 mg, 611.03 µmol, 53.49 µL), followed by the addition of a drop of N,N-dimethylformamide. The reaction mixture was stirred at 20°C for 1 hour. After the reaction was complete, the reaction mixture was directly concentrated under reduced pressure to remove the solvent. The residue was dissolved in dichloromethane (1 mL). To a solution of compound **WX013_12** (86 mg, 244.41 µmol) and N,N-diisopropylethylamine (78.97 mg, 611.03 µmol, 106.43 µL) in dichloromethane (1 mL) was added dropwise the prepared acyl chloride at 0°C. The reaction mixture was then stirred at 20°C for 12 hours. After the reaction was complete, the reaction mixture was concentrated under reduced pressure to remove the solvent. The resulting residue was purified by column chromatography (eluent, petroleum ether/ethyl acetate = 19/1 to 9/1, v/v) to obtain target compound **WX013_13**. ¹H NMR (400 MHz, CDCl₃) δ: 8.11 (s, 1H), 7.58 (d, *J*=5.6 Hz, 1H), 7.42-7.30 (m, 4H), 7.24 (dd, *J*=1.4, 8.2 Hz, 1H), 6.81 (d, *J*=8.4 Hz, 1H), 3.71-3.65 (m, 1H), 3.39 (t, *J*=7.5 Hz, 2H), 3.23 (s, 3H), 2.66-2.47 (m, 2H), 1.71-1.63 (m, 6H), 1.32 (d, *J*=2.4 Hz, 9H), 0.96 (d, *J*=7.2 Hz, 3H).

### Step 13: Synthesis of compound WX013

Compound **WX013_13** (105 mg, 174.86 µmol) was dissolved in a solution of hydrochloric acid in ethyl acetate (4 M, 4 mL) at room temperature under nitrogen atmosphere. The reaction system was stirred at 20°C for 2 hours. After the reaction was complete, the reaction mixture was directly concentrated under reduced pressure to remove the solvent. The residue was purified by preparative HPLC (mobile phase: acetonitrile/water; acidic system: 0.04% HCl) to obtain target compound **WX013**. MS-ESI m/z: 544.2 [M+H]⁺. ¹H NMR (400 MHz, DMSO_*d*₆) δ: 11.87 (s, 1H), 9.83 (d, *J*=2.8 Hz, 1H), 7.51-7.41 (m, 4H), 7.38-7.30 (m, 2H), 6.96 (dd, *J*=2.1, 8.3 Hz, 1H), 4.13 (d, *J*=10.5 Hz, 1H), 3.30-3.24 (m, 2H), 3.10 (d, *J*=1.3 Hz, 3H), 2.62-2.55 (m, 1H), 2.52 (d, *J*=1.9 Hz, 1H), 1.60-1.49 (m, 6H), 0.80 (d, *J*=7.0 Hz, 3H).

### Example 14

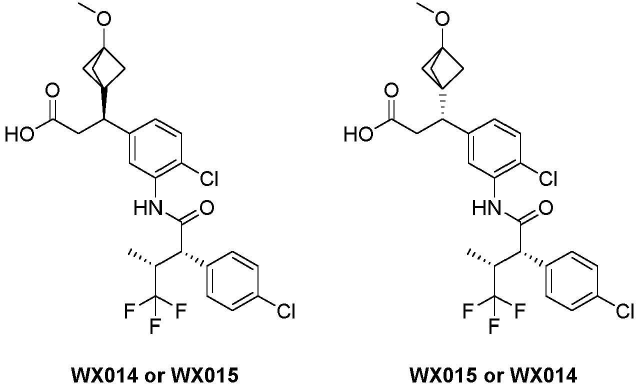

### Step 1: Synthesis of compounds WX014 and WX015

Compound **WX013** was initially separated by chiral column chromatography (column type: DAICEL CHIRALPAK IC (250 mm × 30 mm, 10 µm); mobile phase: A (CO₂) and B (isopropanol containing 0.1% ammonia); gradient: B% = 30% to 30%, 10 minutes) and then separated by preparative HPLC (mobile phase: acetonitrile/water; acidic system: 0.04% HCl) to obtain compounds **WX014** and **WX015,** respectively.

SFC analysis method: column type: (S,S)-WHELK-O1, 50 × 4.6 mm I.D., 3.0 µm; mobile phase: A: hexane, B: [ethanol (containing 0.1% TFA)], gradient: B%: 5% to 50%, 6 minutes.

**WX014** (retention time: 3.051 min): MS-ESI m/z: 544.2 [M+H]⁺. ee value: 100%. ¹H NMR (400 MHz, DMSO_*d*₆) δ: 12.11 (s, 1H), 9.83 (s, 1H), 7.50-7.42 (m, 4H), 7.37-7.31 (m, 2H), 6.96 (dd, *J*=2.0, 8.3 Hz, 1H), 4.13 (d, *J*=10.5 Hz, 1H), 3.30-3.23 (m, 2H), 3.10 (s, 3H), 2.62-2.55 (m, 1H), 2.47-2.44 (m, 1H), 1.64-1.43 (m, 6H), 0.81 (d, *J*=7.2 Hz, 3H).

**WX015** (retention time: 3.194 min): MS-ESI m/z: 544.2 [M+H]⁺. ee value: 100%. ¹H NMR (400 MHz, DMSO_*d*₆) δ: 12.11 (s, 1H), 9.82 (s, 1H), 7.52-7.42 (m, 4H), 7.38-7.29 (m, 2H), 6.95 (dd, *J*=2.1, 8.3 Hz, 1H), 4.13 (d, *J*=10.7 Hz, 1H), 3.30-3.24 (m, 2H), 3.09 (s, 3H), 2.61-2.55 (m, 1H), 2.53-2.52 (m, 1H), 1.60-1.48 (m, 6H), 0.80 (d, *J*=7.0 Hz, 3H).

### Example 16

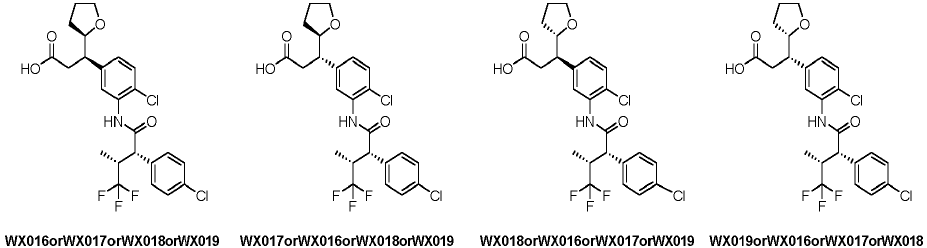

### Synthetic route:

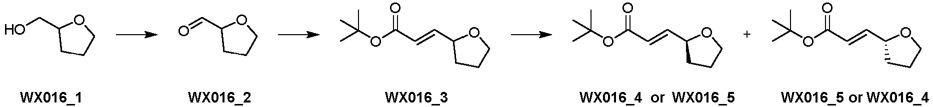

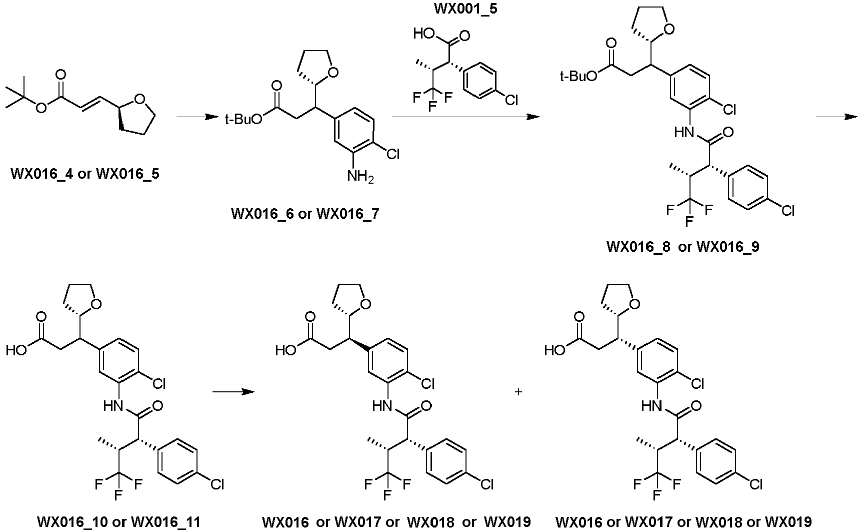

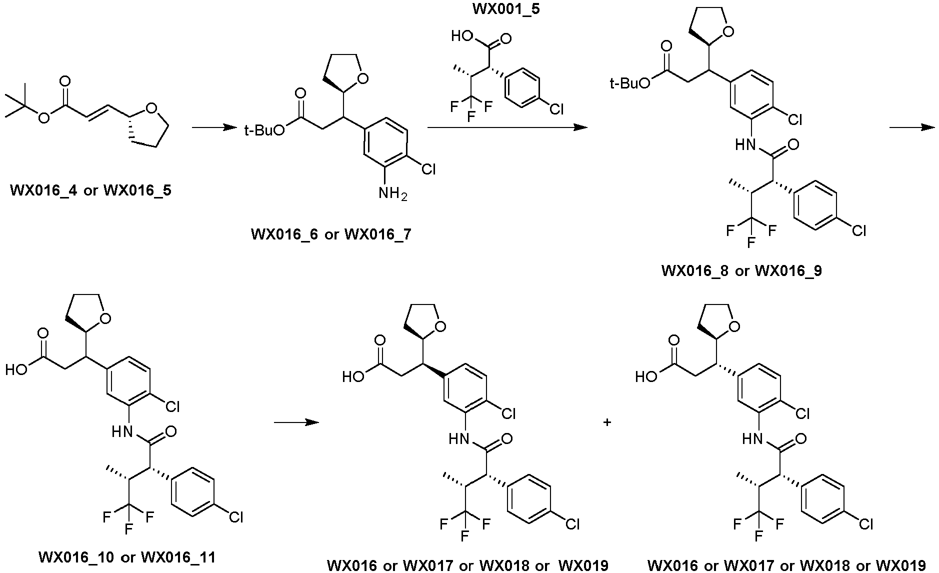

### Step 1: Synthesis of compound WX016_2

**WX016_1** (1.0 g, 9.79 mmol) was dissolved in dichloromethane (10 mL) at room temperature under nitrogen atmosphere, and the reaction mixture was cooled to 0°C under nitrogen atmosphere. Dess-Martin periodinane (4.98 g, 11.75 mmol) was added thereto in batches. The reaction mixture was warmed to 20°C and stirred for 2 hours. After the reaction was complete, the reaction mixture was added with saturated sodium bicarbonate solution (20 mL) and stirred for 0.5 hours. The phases were then separated, and the organic phase was collected. The aqueous phase was extracted with dichloromethane (10 mL × 2). The organic phases were combined, dried over anhydrous sodium sulfate, and filtered. The filtrate was concentrated under reduced pressure to obtain target compound **WX016_2**.

### Step 2: Synthesis of compound WX016_3

tert-Butyl diethylphosphonoacetate (1.01 g, 4.00 mmol) was dissolved in tetrahydrofuran (10 mL) at room temperature under nitrogen atmosphere. The reaction mixture was cooled to 0°C under nitrogen atmosphere, and a 1 M solution of potassium tert-butoxide in tetrahydrofuran (4.39 mL, 4.39 mmol) was added dropwise thereto. After the addition was complete, the reaction mixture was stirred for 0.5 hours. Compound **WX016_2** (400 mg, 4.00 mmol) was then added dropwise thereto. The reaction mixture was stirred at 20°C for 2 hours. After the reaction was complete, the reaction mixture was poured into water (20 mL), and extracted with ethyl acetate (10 mL × 3). The organic phase was washed with saturated brine (30 mL), dried over anhydrous sodium sulfate, filtered, and the filtrate was concentrated under reduced pressure to remove the solvent. The residue was purified by column chromatography (eluent, petroleum ether/ethyl acetate = 1/0 to 9/1, v/v) to obtain target compound **WX016_3**. ¹H NMR (400 MHz, CDCl₃) δ: 6.76-6.85 (m, 1H), 5.89-5.97 (m, 1H), 4.45-4.54 (m, 1H), 3.90-3.98 (m, 1H), 3.80-3.88 (m, 1H), 2.05-2.16 (m, 1H), 1.88-1.97 (m, 2H), 1.66-1.74 (m, 1H), 1.49 (s, 9H).

### Step 3: Synthesis of compounds WX016_4 and WX016_5

Compound **WX016_3** was initially separated by chiral column chromatography (column type: REGIS(S,S)WHELK-O1, (250 mm × 25 mm,10 µm); mobile phase: A (CO₂) and B (isopropanol containing 0.1% ammonia); gradient: B% = 30% to 30%, 10 minutes) to obtain compounds **WX016_4** and **WX016_5**, respectively.

SFC analysis method: column type: WK, 100 × 4.6 mm I.D., 3 µm; mobile phase: A: CO₂, B: [IPA (containing 0.1% IPAm)], gradient: B%: 10% to 50%, 4 minutes.

**WX016_4** (retention time: 0.635 min): ee value: 100%. ¹H NMR (400 MHz, CDCl₃) δ: 6.81 (dd, *J*=5.2, 15.6 Hz, 1H), 5.93 (dd, *J*=1.4, 15.4 Hz, 1H), 4.52-4.46 (m, 1H), 3.98-3.90 (m, 1H), 3.88-3.80 (m, 1H), 2.17-2.07(m, 1H), 1.98-1.91 (m, 2H), 1.75-1.65 (m, 1H), 1.49 (s, 9H)

**WX016_5** (retention time: 0.804 min): ee value: 100%. ¹H NMR (400 MHz, CDCl₃) δ: 6.81 (dd, *J*=5.2, 15.6 Hz, 1H), 5.93 (dd, *J*=1.4, 15.6 Hz, 1H), 4.52-4.46 (m, 1H), 3.97-3.90 (m, 1H), 3.87-3.80 (m, 1H), 2.18-2.08 (m, 1H), 1.99-1.90 (m, 2H), 1.74-1.66 (m, 1H), 1.49 (s, 9H).

### Step 4: Synthesis of compound WX016_6

Chloro(1,5-cyclooctadiene)rhodium(I) dimer (15.54 mg, 31.52 µmol) and 2,2'-bis(diphenylphosphino)-1,1'-binaphthalene (4.51 mg, 7.25 µmol) were dissolved in tetrahydrofuran (3 mL) at room temperature under nitrogen atmosphere. The reaction mixture was stirred at 20°C for 15 minutes. 3-Amino-4-chlorophenylboronic acid pinacol ester (319.70 mg, 1.26 mmol) was dissolved in a mixed solvent of isopropanol (1.5 mL) and tetrahydrofuran (3 mL) at room temperature under nitrogen atmosphere. To the reaction mixture were added compound **WX016_4** (250.00 mg, 1.26 mmol), 1,5-cyclooctadiene (13.64 mg, 126.10 µmol), and potassium hydroxide (84.90 mg, 1.51 mmol). The reaction mixture was heated to 60°C. The previously prepared catalyst was added thereto under nitrogen atmosphere, and the reaction mixture was stirred at 60°C for 12 hours. After the reaction was complete, the reaction mixture was poured into water (10 mL) to quench the reaction, and extracted with ethyl acetate (10 mL × 3). The organic phases were combined, washed with saturated brine (30 mL), dried over anhydrous sodium sulfate, filtered, and the filtrate was concentrated under reduced pressure to remove the solvent. The residue was purified by column chromatography (eluent: 19/1 to 7/3, v/v) to obtain compound **WX016_6.**

### Step 5: Synthesis of compound WX016_8

Compound **WX001_5** (204.59 mg, 767.27 µmol) was dissolved in anhydrous dichloromethane (4 mL) at room temperature under nitrogen atmosphere. The reaction mixture was cooled to 0°C, and oxalyl chloride (243.47 mg, 1.92 mmol, 167.91 µL) was added dropwise thereto. A drop of N,N-dimethylformamide was then added thereto, and the reaction mixture was stirred at 20°C for 1 hour. After the reaction was complete, the reaction mixture was directly concentrated under reduced pressure to remove the solvent. The residue was dissolved in anhydrous dichloromethane (5 mL). To a solution of compound **WX016_6** (250.00 mg, 767.27 µmol) and N,N-diisopropylethylamine (247.91 mg, 1.92 mmol, 334.10 µL) in dichloromethane (2 mL) was added dropwise the prepared acyl chloride at 0°C. The reaction mixture was then stirred at 20°C for 12 hours. After the reaction was complete, the reaction mixture was poured into water (10 mL) to quench the reaction. The phases were separated, and the organic phase was collected. The aqueous phase was extracted with dichloromethane (10 mL). The organic phases were then combined, washed with saturated brine (20 mL), dried over anhydrous sodium sulfate, and filtered. The filtrate was concentrated under reduced pressure to remove the solvent. The resulting residue was purified by column chromatography (eluent, petroleum ether/ethyl acetate = 19/1 to 7/3, v/v) to obtain compound **WX016_8**.

### Step 6: Synthesis of compound WX016_10

Compound **WX016_10** (300.00 mg, 522.23 µmol) was dissolved in ethyl acetate (1 mL) at room temperature under nitrogen atmosphere. A solution of hydrochloric acid in ethyl acetate (4 M, 5 mL) was added thereto, and the reaction mixture was stirred at 20°C for 5 hours. After the reaction was complete, the reaction mixture was directly concentrated under reduced pressure. The crude product was purified by preparative HPLC (mobile phase: acetonitrile/water; acidic system: 0.04% HCl) to obtain target compound **WX016_10.**

### Step 7: Synthesis of compounds WX016 and WX017

Compound **WX016_10** was initially purified by chiral column chromatography (column type: Phenomenex-Cellulose-2 (250 mm × 30 mm, 10 µm); mobile phase: A (CO₂) and B (ethanol containing 0.1% ammonia); gradient: B% = 30% to 30%, 5 minutes) to obtain compounds **WX016** and **WX017,** respectively.

SFC analysis method: column type: Lux Cellulose-2, 50 × 4.6 mm I.D., 3 µm; mobile phase: A: CO₂, B: [isopropanol (containing 0.1% IPAm)], gradient: B%: 5% to 50%, 5 minutes.

**WX016** (retention time: 1.239 min): MS-ESI m/z: 517.9[M+H]⁺. ee value: 99.66%. ¹H NMR (400 MHz, DMSO_*d*₆) δ: 11.93 (s, 1H), 9.81 (s, 1H), 7.49-7.43 (m, 4H), 7.40 (d, *J*=1.9 Hz, 1H), 7.33 (d, *J*=8.3 Hz, 1H), 7.06 (dd, *J*=1.9, 8.3 Hz, 1H), 4.11 (d, *J*=10.6 Hz, 1H), 3.94-3.84 (m, 1H), 3.63-3.47 (m, 2H), 3.42-3.37 (m, 1H), 3.06-2.99 (m, 1H), 2.65-2.59 (m, 2H), 1.90-1.78 (m, 1H), 1.75-1.62 (m, 1H), 1.60-1.48 (m, 1H), 1.45-1.32 (m, 1H), 0.80 (d, *J*=7.1 Hz, 3H).

**WX017** (retention time: 1.542 min): MS-ESI m/z: 517.9[M+H]⁺. ee value: 99.70%. ¹H NMR (400 MHz, DMSO_*d*₆) δ: 11.93 (s, 1H), 9.83 (s, 1H), 7.49-7.43 (m, 4H), 7.41 (d, *J*=1.8 Hz, 1H), 7.36 (d, *J*=8.3 Hz, 1H), 7.08 (dd, *J*=1.9, 8.4 Hz, 1H), 4.12 (d, *J*=10.5 Hz, 1H), 3.86-3.77 (m, 1H), 3.75-3.67 (m, 1H), 3.66-3.57 (m, 1H), 3.44-3.38 (m, 1H), 3.00-2.91 (m, 1H), 2.83-2.76 (m, 1H), 2.45-2.40 (m, 1H), 1.78-1.66 (m, 2H), 1.60-1.50 (m, 1H), 1.41-1.29 (m, 1H), 0.80 (d, *J*=7.1 Hz, 3H).

### Step 8: Synthesis of compound WX016_7

Chloro(1,5-cyclooctadiene)rhodium(I) dimer (15.54 mg, 31.52 µmol) and 2,2'-bis(diphenylphosphino)-1,1'-binaphthalene (4.51 mg, 7.25 µmol) were dissolved in tetrahydrofuran (3 mL) at room temperature under nitrogen atmosphere. The reaction mixture was stirred at 20°C for 15 minutes. 3-Amino-4-chlorophenylboronic acid pinacol ester (319.70 mg, 1.26 mmol) was dissolved in a mixed solvent of isopropanol (1.5 mL) and tetrahydrofuran (3 mL) at room temperature under nitrogen atmosphere. To the reaction mixture were added compound **WX016_5** (250.00 mg, 1.26 mmol), 1,5-cyclooctadiene (13.64 mg, 126.10 µmol), and potassium hydroxide (84.90 mg, 1.51 mmol). The reaction mixture was heated to 60°C. The previously prepared catalyst was added thereto under nitrogen atmosphere, and the reaction mixture was stirred at 60°C for 12 hours. After the reaction was complete, the reaction mixture was poured into water (10 mL) to quench the reaction, and extracted with ethyl acetate (10 mL × 3). The organic phases were combined, washed with saturated brine (30 mL), dried over anhydrous sodium sulfate, filtered, and the filtrate was concentrated under reduced pressure to remove the solvent. The crude product was purified by column chromatography (eluent: 19/1 to 7/3, v/v) to obtain compound **WX016_7**.

### Step 9: Synthesis of compound WX016_9

Compound **WX001_5** (109.66 mg, 411.26 µmol) was dissolved in anhydrous dichloromethane (2 mL) at room temperature under nitrogen atmosphere, and the reaction mixture was cooled to 0°C. To the reaction mixture was added dropwise oxalyl chloride (156.60 mg, 1.23 mmol, 108.00 µL), followed by the addition of a drop of N,N-dimethylformamide. The reaction system was stirred at 0°C for 1 hour. After the reaction was complete, the reaction mixture was directly concentrated under reduced pressure to remove the solvent. The residue was dissolved in anhydrous dichloromethane (2 mL). Compound **WX016_7** (134.00 mg, 411.26 µmol) and N,N-diisopropylethylamine (132.88 mg, 1.03 mmol, 179.08 µL) were sequentially added thereto at 0°C. The reaction mixture was then stirred at 25°C for 12 hours. After the reaction was complete, the reaction mixture was directly concentrated under reduced pressure to remove the solvent. The resulting residue was purified by column chromatography (eluent, petroleum ether/ethyl acetate = 19/1 to 7/3, v/v) to obtain target compound **WX016_9**.

### Step 10: Synthesis of compounds WX018 and WX019

Compound **WX016_9** (174.00 mg, 302.89 µmol) was dissolved in ethyl acetate (1 mL) at room temperature under nitrogen atmosphere. A solution of hydrochloric acid in ethyl acetate (4 M, 5 mL) was added thereto, and the reaction mixture was stirred at 25°C for 1.5 hours. After the reaction was complete, the reaction mixture was directly concentrated under reduced pressure to remove the solvent. The resulting residue was purified by preparative HPLC (mobile phase: acetonitrile/water; acidic system: 0.04% HCl) to obtain target compounds **WX018** and **WX019.**

SFC analysis method: column type: Chiralpak IC-3, 50 × 4.6 mm I.D., 3 µm; mobile phase: A: CO₂, B: [isopropanol (containing 0.1% IPAm)], gradient: B%: 5% to 50%, 3 minutes.

**WX018** (retention time: 1.286 min): MS-ESI m/z: 517.9 [M+H]⁺. ee value: 100%. ¹H NMR (400 MHz, DMSO_*d*₆) δ: 12.03 (s, 1H), 9.81 (s, 1H), 7.49-7.43 (m, 4H), 7.43-7.41 (m, 1H), 7.33 (d, *J*=8.3 Hz, 1H), 7.06 (dd, *J*=2.0, 8.4 Hz, 1H), 4.12 (d, *J*=10.6 Hz, 1H), 3.94-3.86 (m, 1H), 3.62-3.47 (m, 2H), 3.41-3.34 (m, 1H), 3.07-2.98 (m, 1H), 2.63-2.60 (m, 1H), 2.57-2.53 (m, 1H), 1.90-1.78 (m, 1H), 1.75-1.62 (m, 1H), 1.60-1.49 (m, 1H), 1.45-1.34 (m, 1H), 0.80 (d, *J*=7.1 Hz, 3H).

**WX019** (retention time: 1.226 min): MS-ESI m/z: 517.9 [M+H]⁺. ee value: 92.60%. ¹H NMR (400 MHz, DMSO_*d*₆) δ: 11.94 (s, 1H), 9.83 (s, 1H), 7.51-7.43 (m, 4H), 7.42-7.39 (m, 1H), 7.38-7.34 (m, 1H), 7.08 (dd, *J*=2.0, 8.4 Hz, 1H), 4.12 (d, *J*=10.6 Hz, 1H), 3.85-3.77 (m, 1H), 3.75-3.67 (m, 1H), 3.65-3.58 (m, 1H), 3.41-3.35 (m, 1H), 3.00-2.92 (m, 1H), 2.80 (dd, *J*=4.6, 15.9 Hz, 1H), 2.46-2.40 (m, 1H), 1.79-1.66 (m, 2H), 1.56 (dd, *J*=6.0, 13.5 Hz, 1H), 1.41-1.30 (m, 1H), 0.80 (d, *J*=7.1 Hz, 3H).

### Bioassay:

### Test example 1: In vitro activity assay

### I. cGMP expression test based on InCap cells

### 1. Experimental steps

### 1) Solution preparation

### ·10% BSA (bovine serum albumin)

10g of BSA was dissolved in 100 mL of double-distilled water (ddH₂O) to obtain 10% BSA.

### ·10 mM ODQ stock solution

1 mg of ODQ powder was weighed and dissolved in 534 µL of DMSO to prepare 10 mM ODQ solution. The solution was aliquoted and stored in a -20°C refrigerator.

### ·Washing buffer (50 mL)

| **Volume** | **Final concentration** |
|---|---|
| 49 mL of Earls balanced salt solution (EBSS) | 1x |
| 500 µL of 1 M hydroxyethyl piperazine ethanesulfonic acid (HEPES) | 10 mM |
| 250 µL of 10% BSA stabilizer | 0.05% |
| 250 µL of 1 M MgCl₂ | 5 mM |

### ·Assay buffer (50 mL)

| **Volume** | **Final concentration** |
|---|---|
| 48.95 mL of Earls balanced salt solution (EBSS) | 1x |
| 500 µL of 1 M hydroxyethyl piperazine ethanesulfonic acid (HEPES) | 10 mM |
| 250 µL 10% BSA | 0.05% |
| 50 µL of 500 mmol/L isobutylmethylxanthine (IBMX) | 0.5 mM |
| 250 µL of 1 M MgCl₂ | 5 mM |

### ·Detection buffer

a) 50 µL of cGMP-D2 (D2-labeled cyclic guanosine monophosphate) was added to 1 mL of lysis buffer and mixed thoroughly.
b) 50 µL of anti-cGMP cryptate (Eu³⁺ cryptate-labeled anti-cyclic guanosine monophosphate antibody) was added to 1 mL of lysis buffer and mixed thoroughly.

### 2) Compound dilution

(1) The compound was diluted to a concentration of 10 mM using DMSO.
(2) A serial dilution of the compound was performed, resulting in 10 different concentration levels of each compound. 100 nL of each diluted compound was added to a 96-well plate.

### 3) Preparation of LNCap cells

(1) LNCap culture medium: RPMI1640 supplemented with 10% fetal bovine serum (FBS) and 1% double antibody
(2) The phosphate-buffered saline (PBS), trypsin, and culture medium for use in cell passage were preheated in a 37°C water bath.
(3) Cells were removed from a 37°C, 5% CO₂ incubator, and the old culture medium was removed from the culture flask using a pipette.
(4) 5 mL of PBS was pipetted into the flask for rinsing the cells, and then the liquid was discarded.
(5) 3 mL of trypsin was pipetted into the flask. After shaking, the liquid was discarded, and the flask was returned to the incubator.
(6) Approximately 2 minutes later, the flask was retrieved, and cell detachment was observed. 9 mL of culture medium was pipetted into the culture flask and mixed by repetitive pipetting. The cell suspension was then transferred to a 50 mL centrifuge tube.
(7) 0.7 mL of cell suspension was pipetted into a counting chamber, and cell counting was performed using the ViCell XR. The remaining cells were centrifuged at 1000 rpm for 5 minutes, and the supernatant was discarded.
(8) 10 mL of washing buffer was added to wash the cells, followed by another centrifugation at 1000 rpm for 5 minutes, after which the supernatant was discarded.
(9) Assay buffer was added, and the cell concentration was adjusted to 3 × 10⁶/mL.

### 4) Preparation and addition of ODQ solution

(1) 10 mM ODQ stock solution was diluted at a ratio of 1:1000 and added to the cell solution.
(2) After thorough mixing, the solution was added to a microplate at 10 µL per well.

### 5) Preparation of cGMP standard curve

(1) 1 mM cGMP stock solution was diluted to 10 µM using assay buffer. 11 concentration gradients were created through 4-fold serial dilutions.
(2) The diluted cGMP was added to the microplate at 10 µL per well.

### 6) Addition of assay reagents and plate reading

(1) cGMP-D2 was transferred to a 384-well plate at 5 µL per well. Anti-cGMP cryptate was transferred to a 96-well plate at 5 µL per well. Centrifugation was performed at 1500 rpm for 1 minute.
(2) Incubation was carried out at room temperature for 1 hour.
(3) The plates were read using envision at 665/615 wavelengths.

### 7) Data analysis

(1) cGMP standard curve: A standard curve was generated using GraphPad Prism, based on the ratio of cGMP concentration to the 665/615 wavelength values.
(2) Conversion of HTRF (homogeneous time-resolved fluorescence) ratio (665/615) to cGMP concentration: In GraphPad Prism, the HTRF ratio (665/615) was copied to the ratio column of the cGMP standard curve. The analysis "Log inhibitor vs response-variable slope" was run, and the "interpolate" option was selected to convert the HTRF ratio (665/615) into cGMP concentration.
(3) Compound activation curve: A curve was generated using the "Log agonist vs response-variable slope" analysis method in GraphPad Prism, based on the converted cGMP concentration and compound concentration.

**Table 1: MEC values of the compounds of the present disclosure for sGC stimulatory activity**

| **Compound** | **MEC (nM)** | **EC50 (nM)** |
|---|---|---|
| WX001 | 4.20 | 6.62 |
| WX002 | 2.20 | 1.76 |
| WX003 | NA | 27.72 |
| WX004 | 0.61 | 3.74 |
| WX005 | 0.27 | 2.06 |
| WX006 | 7.92 | 33.60 |
| WX007 | 0.65 | 3.28 |
| WX008 | 0.95 | 4.14 |
| WX009 | 33.06 | 28.96 |
| WX010 | 1.76 | 2.16 |
| WX011 | 1.39 | 1.75 |
| WX012 | 25.92 | 66.08 |
| WX013 | 1.22 | 3.50 |
| WX014 | 0.42 | 1.65 |
| WX015 | 0.82 | 16.71 |
| WX016 | 27.85 | 145.0 |
| WX019 | 9.83 | 81.24 |

| | | |
|---|---|---|
| MEC: Minimum effective concentration required to stimulate cGMP production in LNCap cells (greater than three times the baseline value). NA indicates not measured. Experimental conclusion: The compounds of the present disclosure are effective in stimulating sGC, thereby increasing cGMP levels. | | |

### Test example 2: In vitro hepatocyte metabolic stability study

### 1. Experimental objective: The aim of the study was to assess the stability of the compounds in hepatocytes of different species.

### 2. Experimental steps:

A variety of 96-well precipitation plates were prepared and labeled as T0, T15, T30, T60, T90, T0-MC, T90-MC, and blank matrix, respectively. Revival medium and incubation medium were taken in advance and pre-warmed in a 37°C water bath. Hepatocytes of different species, stored in a liquid nitrogen tank, were taken and immediately immersed in a 37°C water bath for approximately 90 seconds. Once partially thawed, cells were transferred to centrifuge tubes containing 40 mL of revival medium, and gently inverted to resuspend the cells in the revival medium. At room temperature, cells were centrifuged at 100 × g for 5 minutes. The supernatant was discarded, and the hepatocytes were resuspended in an appropriate volume of incubation medium. Viability was calculated using trypan blue staining. 198 µL of hepatocyte suspension (0.51×10⁶ cells/mL) was added to pre-warmed incubation plates. For the control group, 198 µL of hepatocyte-free incubation medium was added to T0-MC and T120-MC plates. All plates were pre-incubated for 10 minutes in a 37°C incubator. 2 µL of test and control compound working solutions were then added, mixed thoroughly, and the incubation plates were immediately placed in a shaker within the incubator. The timer was initiated to start the reaction. Two duplicate samples were prepared at each time point for each compound. Incubation conditions were set at 37°C, saturated humidity, and 5% CO₂. In the test system, the test compound had a final concentration of 1 µM, the control compound had a final concentration of 3 µM, the hepatocyte had a final concentration of 0.5×10⁶ cells/mL, and the total organic solvent had a final concentration of 0.96%, of which DMSO had a final concentration of 0.1%. At the end of the incubation at the corresponding time point, the incubation plate was removed, and 25 µL of the compound and control compound-cell mixture was transferred to a sample plate containing 125 µL of stop solution (acetonitrile solution containing 200 ng/mL of tolbutamide and Labetalol). For the blank sample plate, 25 µL of hepatocyte-free incubation medium was directly added. All sample plates were sealed and shaken on the shaker at 600 rpm for 10 minutes, and then centrifuged at 3220 × g for 20 minutes. The supernatant of test and control samples was diluted with ultrapure water at a ratio of 1:3. All samples were mixed well and analyzed using LC/MS/MS methods.

The experimental results are shown in Table 2.

**Table 2: Stability of the compounds of the present disclosure in hepatocytes of different species**

| Species | Test item | WX005 | WX015 |
|---|---|---|---|
| Mouse | T_{1/2}-min | 27.0 | 85.2 |
| | CL_{int (liver)} (mL/min/kg) | 610.4 | 193.3 |
| Rat | T_{1/2}-min | 41.9 | 78.2 |
| | CL_{int (liver)} (mL/min/kg) | 154.7 | 82.9 |
| Human | T_{1/2}-min | 77.6 | 154.7 |
| | CL_{int (liver)} (mL/min/kg) | 49.7 | 24.9 |

| | | | |
|---|---|---|---|
| T_{1/2}: half-life; CLᵢₙₜ₍ₗᵢᵥₑᵣ₎: hepatic clearance Experimental conclusion: The compounds of the present disclosure exhibit good stability in human-derived hepatocytes, with moderate hepatic clearance and half-life. | | | |

### Test example 3: In vivo pharmacokinetic property study

Experimental objective: The aim of this study was to determine the pharmacokinetic parameters of the compound in male Sprague Dawley rats.

### Experimental materials:

### Sprague Dawley rats (male, 200 to 300g, 7 to 9 weeks old, procured from Shanghai SLAC)

### Experimental methods:

For this project, four male Sprague Dawley rats were used. Two rats in one group were administered the compound intravenously at a dosage of 2 mg/kg and a concentration of 0.4 mg/mL. Two rats in another group were administered the compound orally at a dosage of 10 mg/kg and a concentration of 1 mg/mL. Plasma samples were collected 0.083 (intravenous group only), 0.25, 0.5, 1, 2, 4, 6, 8, and 24 hours after administration. The collected samples were then analyzed using LC-MS/MS and data were collected. The collected analytical data were processed using Phoenix WinNonlin 6.3 software to calculate relevant pharmacokinetic parameters.

The experimental results are shown in Table 3.

**Table 3: Results of in vivo pharmacokinetic experiments**

| Test sample | | **WX005** | **WX015** |
|---|---|---|---|
| iv (2 mg/kg) | **Cl (mL/min/kg)** | 5.47 | 6.7 |
| | **V_{dss} (L/kg)** | 1.27 | 0.67 |
| | **T_{1/2} (h)** | 3.83 | 2.38 |
| | **AUC (h*nmol/L)** | 11228 | 9147 |
| po (10 mg/kg) | **T_{1/2} (h)** | 6.92 | 2.72 |
| | **AUC (h*nmol/L)** | 17423 | 36252 |
| | %F | 42.1% | 79.4% |

| | | | |
|---|---|---|---|
| **[0282] Conclusion:** The compounds of the present disclosure exhibit favorable clearance, half-life, and oral bioavailability by gavage. | | | |

## Claims

1. A compound of formula (II) or a pharmaceutically acceptable salt thereof, 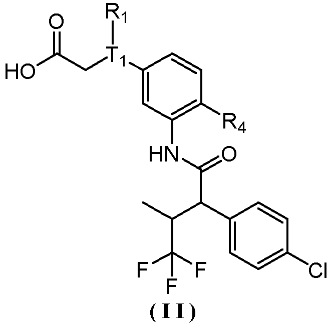 wherein
R₁ is selected from 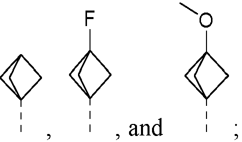
R₄ is selected from halogen and C₁₋₃ alkyl;
T₁ is selected from CH and N.

2. The compound or the pharmaceutically acceptable salt thereof according to claim 1, wherein R₄ is selected from F, Cl, and methyl.

3. The compound or the pharmaceutically acceptable salt thereof according to claim 1 or 2, wherein the compound is selected from: 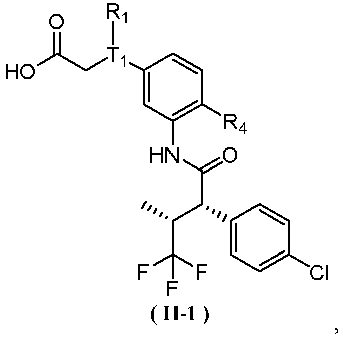 wherein R₁, R₄, and T₁ are as defined in claim 1 or 2.

4. The compound or the pharmaceutically acceptable salt thereof according to claim 1, wherein the compound is selected from:

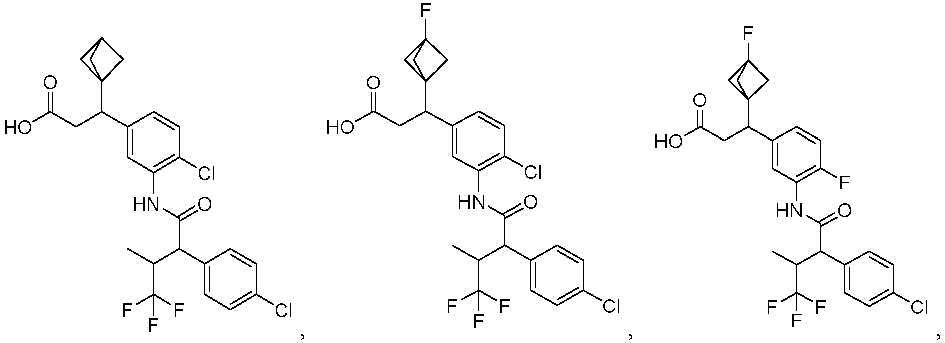

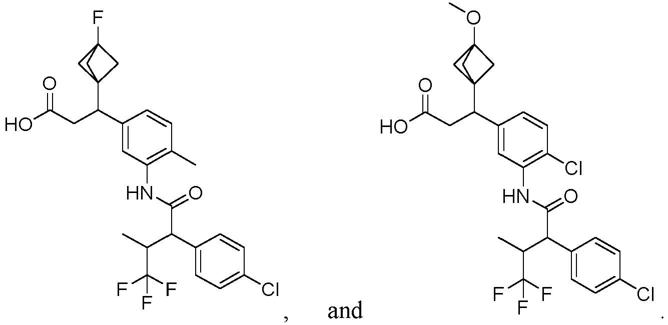

5. The compound or the pharmaceutically acceptable salt thereof according to claim 4, selected from:

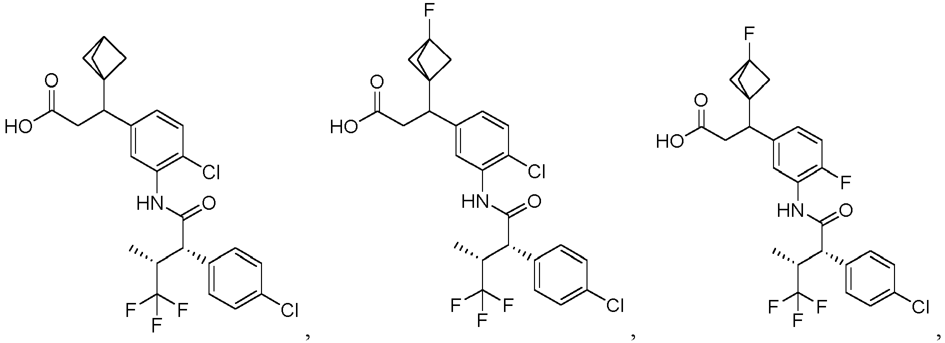

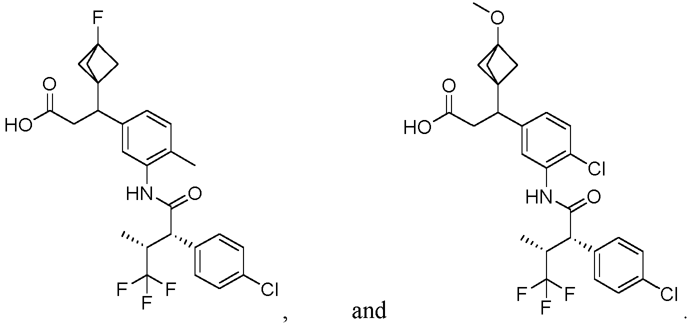

6. The compound or the pharmaceutically acceptable salt thereof according to claim 5, selected from: 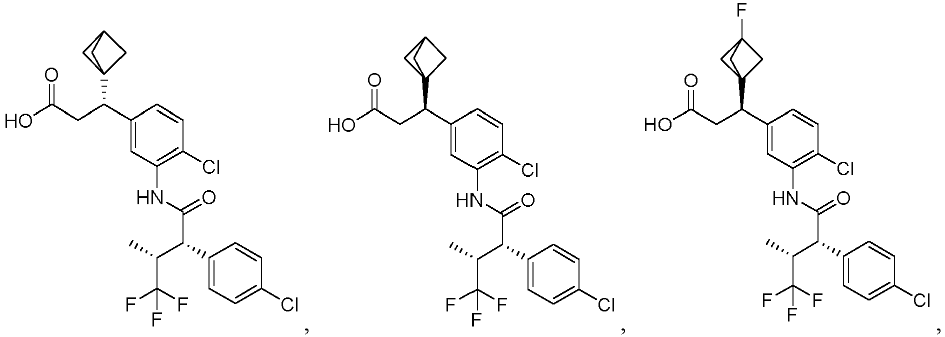 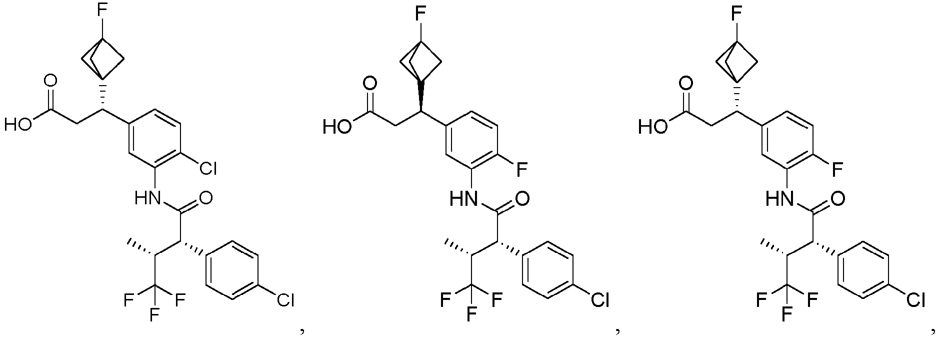 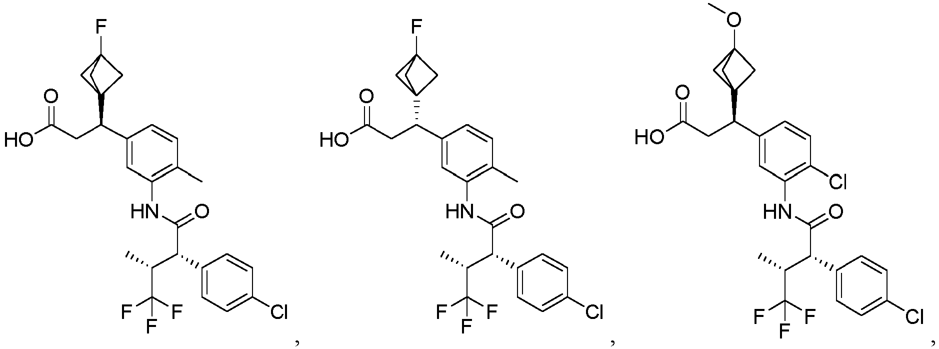 and

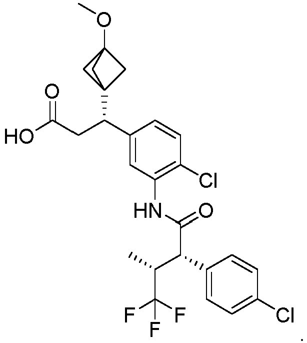

7. The compound or the pharmaceutically acceptable salt thereof according to any one of claims 1 to 6 for use in treating chronic kidney disease.

## Patentansprüche

1. Verbindung der Formel (II) oder ein pharmazeutisch akzeptables Salz davon, 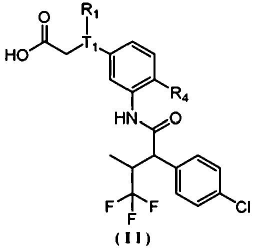 wobei
R₁ ausgewählt ist aus 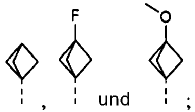
R₄ ausgewählt ist aus Halogen und C₁-₃-Alkyl;
T₁ ausgewählt ist aus CH und N.

2. Verbindung oder das pharmazeutisch akzeptable Salz davon nach Anspruch 1, wobei R₄ ausgewählt ist aus F, Cl und Methyl.

3. Verbindung oder das pharmazeutisch akzeptable Salz davon nach Anspruch 1 oder 2, wobei die Verbindung ausgewählt ist aus: 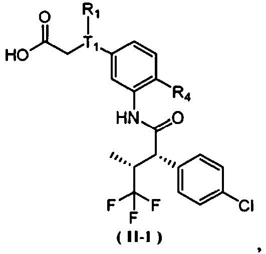 wobei R₁, R₄, und T₁ wie in Anspruch 1 oder 2 definiert sind.

4. Verbindung oder das pharmazeutisch akzeptable Salz davon nach Anspruch 1, wobei die Verbindung ausgewählt ist aus:

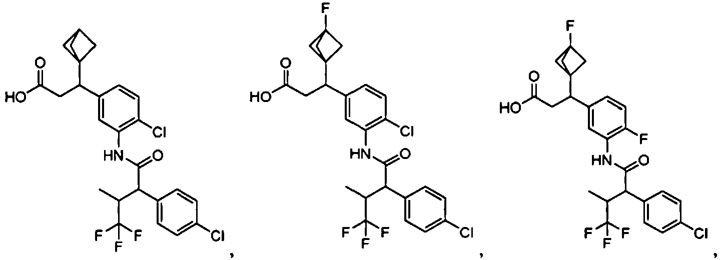

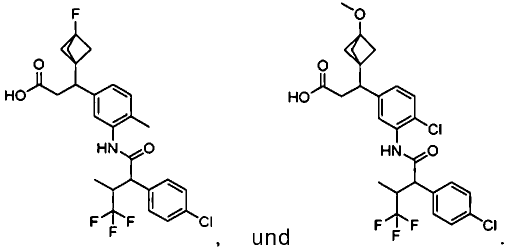

5. Verbindung oder das pharmazeutisch akzeptable Salz davon nach Anspruch 4, ausgewählt aus:

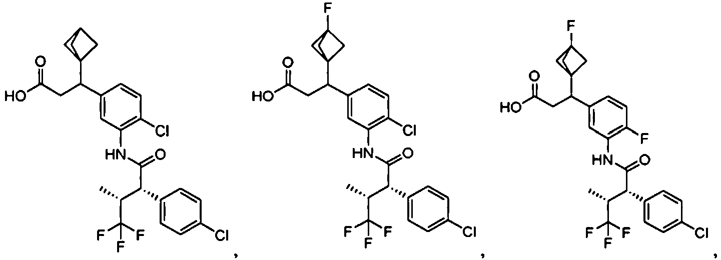

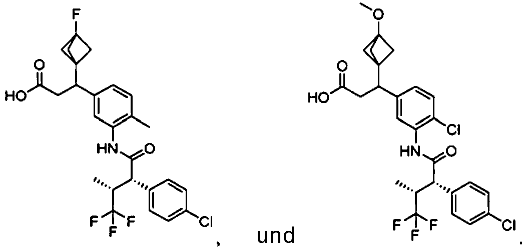

6. Verbindung oder das pharmazeutisch akzeptable Salz davon nach Anspruch 5, ausgewählt aus: 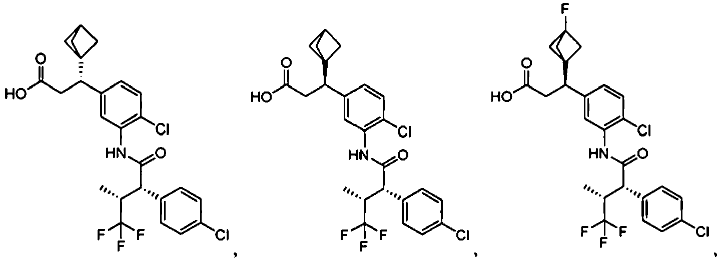 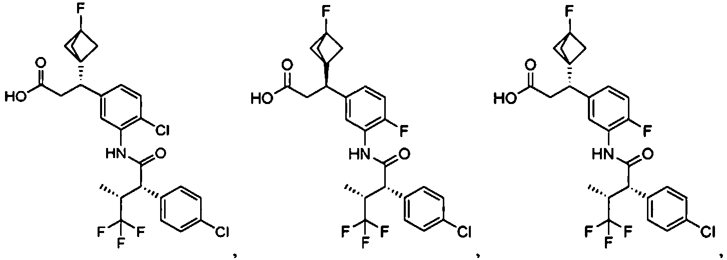 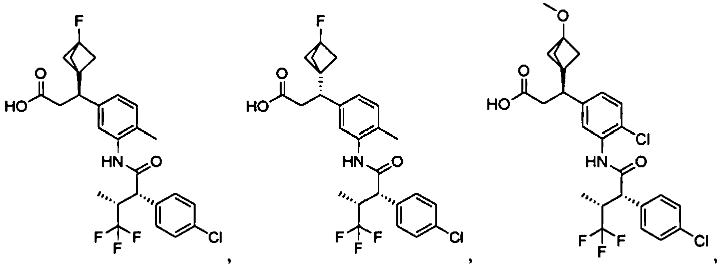 und

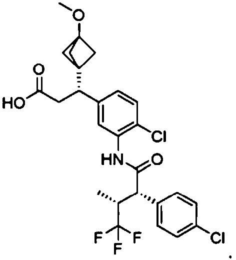

7. Verbindung oder das pharmazeutisch akzeptable Salz davon nach einem der Ansprüche 1 bis 6 zur Verwendung bei der Behandlung einer chronischen Nierenerkrankung.

## Revendications

1. Composé de formule (II) ou sel pharmaceutiquement acceptable de celui-ci, 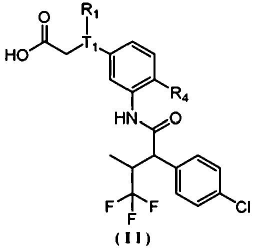 dans lequel
R₁ est sélectionné parmi, 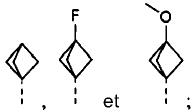
R₄ est sélectionné parmi un halogène et un alkyle C₁-₃ ;
T₁ est sélectionné parmi CH et N.

2. Composé ou sel pharmaceutiquement acceptable de celui-ci selon la revendication 1, dans lequel R₄ est sélectionné parmi F, CI, et un méthyle.

3. Composé ou sel pharmaceutiquement acceptable de celui-ci selon la revendication 1 ou la revendication 2, dans lequel le composé est sélectionné parmi : 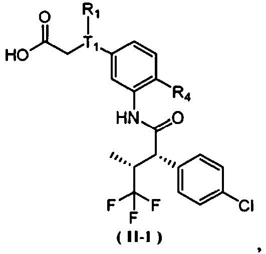 dans lequel R₁, R₄, et T₁ sont tels que définis dans la revendication 1 ou la revendication 2.

4. Composé ou sel pharmaceutiquement acceptable de celui-ci selon la revendication 1, dans lequel le composé est sélectionné parmi :

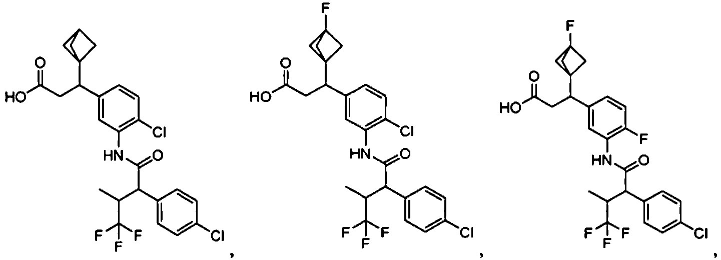

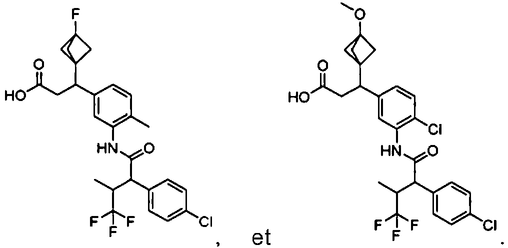

5. Composé ou sel pharmaceutiquement acceptable de celui-ci selon la revendication 4, sélectionné parmi :

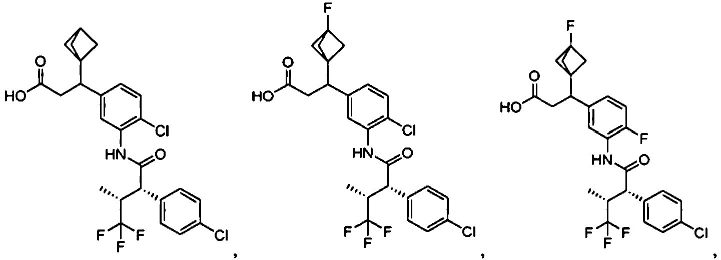

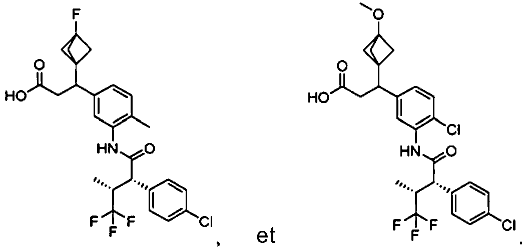

6. Composé ou sel pharmaceutiquement acceptable de celui-ci selon la revendication 5, sélectionné parmi : 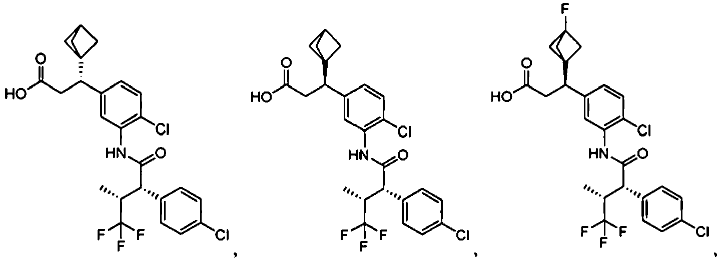 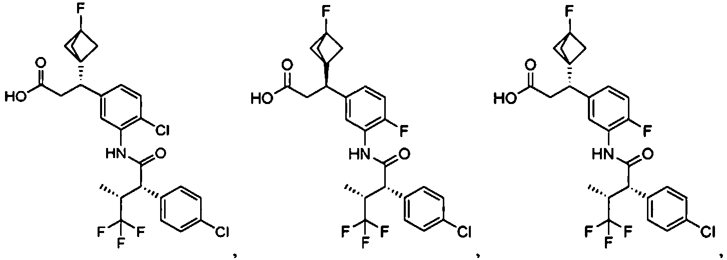 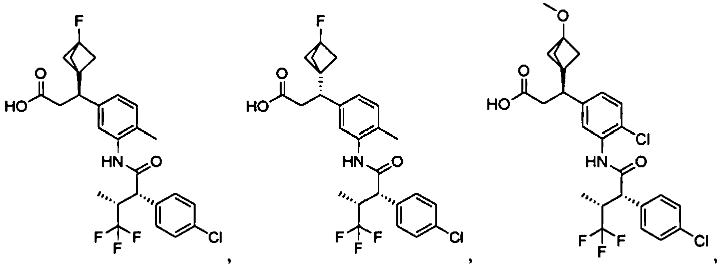 et

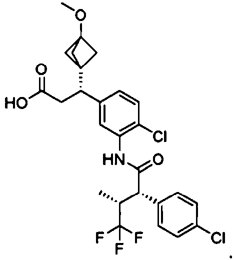

7. Composé ou sel pharmaceutiquement acceptable de celui-ci selon l'une quelconque des revendications 1 à 6 pour une utilisation dans le traitement de maladies rénales chroniques.
